(19) Europäisches Patentamt
European Patent Office
Office européen des brevets

(11) **EP 3 728 624 B1**

(12) **FASCICULE DE BREVET EUROPEEN**

(45) Date de publication et mention
de la délivrance du brevet:
**16.07.2025 Bulletin 2025/29**

(21) Numéro de dépôt: **18842789.2**

(22) Date de dépôt: **20.12.2018**

(51) Classification Internationale des Brevets (IPC):
**G01N 21/31** (2006.01)  **C12Q 1/04** (2006.01)

(52) Classification Coopérative des Brevets (CPC):
**C12Q 1/04; G01N 21/31; G06V 10/58; G06V 20/698**

(86) Numéro de dépôt international:
**PCT/FR2018/053436**

(87) Numéro de publication internationale:
**WO 2019/122734 (27.06.2019 Gazette 2019/26)**

(54) **PROCEDE D'IDENTIFICATION D'UNE LEVURE OU D'UNE BACTERIE**

VERFAHREN ZUR IDENTIFIZIERUNG VON HEFE ODER BAKTERIEN

METHOD FOR IDENTIFYING YEAST OR BACTERIA

(84) Etats contractants désignés:
AL AT BE BG CH CY CZ DE DK EE ES FI FR GB GR HR HU IE IS IT LI LT LU LV MC MK MT NL NO PL PT RO RS SE SI SK SM TR

(30) Priorité: **21.12.2017 FR 1762828**

(43) Date de publication de la demande:
**28.10.2020 Bulletin 2020/44**

(73) Titulaire: **bioMérieux**
**69280 Marcy l'Etoile (FR)**

(72) Inventeurs:
• **LALLEMAND, Jordane**
**34750 Villeneuve les Maguelone (FR)**
• **LEROUX, Denis**
**01600 Trevoux (FR)**
• **PETIT, Manuel**
**38000 Grenoble (FR)**

(74) Mandataire: **bioMérieux PI Groupement mandataires**
**bioMérieux**
**69280 Marcy l'Etoile (FR)**

(56) Documents cités:
EP-A1- 3 257 947      WO-A2-2010/077304
US-A1- 2012 135 454

• HUISUNG KIM ET AL: "Development of a multispectral light-scatter sensor for bacterial colonies", JOURNAL OF BIOPHOTONICS, vol. 10, no. 5, 14 July 2016 (2016-07-14), DE, pages 634 - 644, XP055454322, ISSN: 1864-063X, DOI: 10.1002/jbio.201500338
• GUILLEMOT MATHILDE ET AL: "Hyperspectral imaging for presumptive identification of bacterial colonies on solid chromogenic culture media", PROCEEDINGS OF SPIE; [PROCEEDINGS OF SPIE ISSN 0277-786X VOLUME 10524], SPIE, US, vol. 9887, 27 April 2016 (2016-04-27), pages 98873L - 98873L, XP060069290, ISBN: 978-1-5106-1533-5, DOI: 10.1117/12.2229761
• BOSOON PARK ET AL: "Hyperspectral microscope imaging methods to classify gram-positive and gram-negative foodborne pathogenic bacteria", TRANSACTIONS OF THE AMERICAN SOCIETY OF AGRICULTURAL ENGINEERS, AMERICAN SOCIETY OF AGRICULTURAL ENGINEERS. ST.JOSEPH, MI, US, vol. 58, no. 1, 1 January 2015 (2015-01-01), pages 5 - 16, XP008182213, ISSN: 0001-2351, DOI: 10.13031/TRANS.58.10832

- GRAUS MATTHEW S ET AL: "Hyperspectral fluorescence microscopy detects autofluorescent factors that can be exploited as a diagnostic method for species differentiation", JOURNAL OF BIOMEDICAL OPTICS, SPIE, 1000 20TH ST. BELLINGHAM WA 98225-6705 USA, vol. 22, no. 1, 1 January 2017 (2017-01-01), pages 16002, XP060082688, ISSN: 1083-3668, [retrieved on 20170105], DOI: 10.1117/1.JBO.22.1.016002

- J. D. WALSH ET AL: "Rapid Intrinsic Fluorescence Method for Direct Identification of Pathogens in Blood Cultures", MBIO, vol. 4, no. 6, 19 November 2013 (2013-11-19), pages e00865 - 13, XP055250247, DOI: 10.1128/mBio.00865-13

## Description

**DOMAINE DE L'INVENTION**

L'invention a trait au domaine de l'analyse microbiologique, et en particulier de la caractérisation de microorganismes, notamment l'identification de levures et de bactéries, et dans le cadre de ces dernières l'identification de leur type de Gram et de leur caractère fermentant ou non.

De manière avantageuse, l'invention s'applique à l'analyse d'une image hyperspectrale ou multispectrale d'une colonie bactérienne ou de levure ayant poussé dans un milieu nutritif non chromogène, non fluorogène et dépourvu de colorant.

**ETAT DE LA TECHNIQUE**

Dans le domaine des microorganismes pathogènes, la caractérisation d'un microorganisme consiste préférentiellement à identifier son espèce et sa sensibilité à un agent antimicrobien, (ou « antibiogramme »), afin de déterminer un traitement pour le patient infecté par ce microorganisme. Pour ce faire, un processus microbiologique complexe est usuellement mis en œuvre en laboratoire, processus qui nécessite le plus souvent la connaissance préalable d'autres propriétés du microorganisme, notamment son règne (e.g. levure ou bactérie), et dans le cadre bactérien son type de Gram ou son caractère fermentaire ou non. En effet, ces informations permettent notamment de choisir un milieux de culture ou un type d'agents antimicrobien adaptés au microorganisme afin de déterminer, *in fine*, son espèce ou son antibiogramme. Par exemple, le choix d'une galerie d'identification de microorganismes API® commercialisée par la demanderesse se fonde sur la connaissance du règne du microorganisme (e.g. levure vs bactérie) ou du type de Gram de la souche bactérienne à identifier. De même la détermination de l'antibiogramme d'une souche bactérienne par le système Vitek ® 2 commercialisé par la demanderesse se fonde sur le choix d'une carte en fonction du type de Gram et du caractère fermentant ou non de ladite souche. Il est également possible de citer l'identification par spectrométrie de masse MALDI-TOF employant une matrice différente selon que le microorganisme à identifier est une levure ou une bactérie. Ainsi connaître au plus tôt ces informations permet d'optimiser le processus microbiologique, notamment en accélérant ce dernier ou en réduisant le nombre de consommables employés.

La connaissance de ces propriétés permet également de réduire les faux positifs d'identification de souches bactériennes. A titre d'exemple, dans le cadre du milieu ChomID® Elite Medium commercialisé par la Demanderesse, la connaissance du caractère fermentant de la souche bactérienne testée robustifie l'identification des salmonelles. En particulier, une salmonelle, bactérie fermentante, et une *Pseudomonas*, bactérie non fermentante, font toutes deux virer le substrat chromogène. Savoir si la bactérie est non fermentante permet ainsi d'écarter simplement la salmonelle sans test microbiologique supplémentaire.

Outre la caractérisation d'un microorganisme en vue d'orienter le processus microbiologique en laboratoire, ces informations ont également une utilité clinique. Notamment, la classification de Gram d'une souche de bactérie permet de caractériser la paroi celle-ci, par exemple son pourcentage en peptidoglycane, et est utilisé dans la taxonomie des bactéries ou pour évaluer en première approximation leur sensibilité aux antibiotiques. On distingue ainsi deux types de bactérie, à savoir les bactéries à Gram « positif » et les bactéries à Gram « négatif ». De même on observe que les bactéries non fermentantes, i.e. les bactéries incapables de cataboliser le glucose, occupent une place particulière chez les bactéries pathogènes. En effet, elles ont un niveau de résistance naturelle aux antibiotiques élevée et sont impliquées dans nombre d'infections nosocomiales. A titre d'exemple, on peut citer *Pseudomonas aeruginosa* et *Acinetobacter*. Connaître rapidement le caractère fermentant ou non d'une bactérie permet ainsi d'orienter plus efficacement l'antibiothérapie de première intention et de ralentir la diffusion de souches multirésistantes.

Historiquement, chacune des propriétés cités précedemment (règne, Gram et fermantant) est obtenu par une technique dédiée. Par exemple, le type de Gram d'une souche bactérienne était déterminé par une technique manuelle dite de « coloration de Gram », qui comprend un nombre important d'étapes manuelles (fixation, coloration, mordançage, lavage, sur-coloration...), et donc longue à mettre en œuvre. Diverses techniques ont donc été mises au point pour automatiser la détection du type de Gram des bactéries, afin notamment de traiter un nombre important d'échantillons. Toutefois, ces techniques continuent pour l'essentiel à modifier la réponse électromagnétique des bactéries ou de leur milieu pour rendre leur Gram aisément observable. En particulier, un premier type de technique consiste à automatiser la coloration de la membrane bactérienne sur des lamelles de microscope, mais l'étape de décision finale concernant le type de Gram est toujours réalisée par un technicien qui observe les lamelles au microscope. Ce type de technique n'est donc pas entièrement automatisée, et par ailleurs difficilement automatisable. En effet, la différence de couleurs entre les bactéries de Gram positif et les bactéries de Gram négatif peut être subtil, expliquant pourquoi l'intervention d'un technicien de laboratoire est encore nécessaire. Un deuxième type de technique consiste à mettre des bactéries en présence d'un substrat qui se dégrade par une réaction enzymatique initiée par les peptidoglycanes des membranes des bactéries. Cette réaction produit des chromophores ou des fluorophores dont la concentration est une indication du Gram.

On parle usuellement de « marquage » chromogène ou fluorogène des bactéries. Si ce type de techniques de l'état de l'art est automatisable, par exemple en mesurant l'intensité lumineuse des chromophores/fluorophores à l'aide d'un dispositif approprié (e.g. spectromètre/fluoromètre) puis en comparant informatiquement l'intensité mesurée à des valeurs de seuils prédéfinies, elles supposent néanmoins la conception de substrats chromophores ou fluorogènes particuliers, souvent couteux. En outre, quelle que soit la technique employée, les bactéries subissent une modification de leur état naturel (e.g. elles comprennent des colorants, ont fixé des marqueurs chromogènes ou fluorescentes, etc.), et ne sont donc plus utilisables pour des tests de caractérisations ultérieurs (e.g. la détermination d'un antibioGramme).

Concernant la détermination du caractère fermentant ou non d'une bactérie, elle est usuellement mise en œuvre l'emploi de milieu chromogènes qui changent de couleur en fonction du caractère fermentant ou non de la souche bactérienne testée. Par exemple, le test de « Kligler-Hajna » consiste à faire pousser la souche sur un milieu de culture comprenant un indicateur colorimétrique changeant de couleur en fonction du pH, du lactose, du glucose du thiosulfate et des ions ferreux. Ce milieu détecte le caractère fermentant de la bactérie par la catabolisation du glucose qui se traduit par virage colorimétrique de l'indicateur de pH. On peut également citer les milieux de test de l'activité de l'estérase de tributyrine de la souche bactérienne qui permettent de caractériser les bactéries à Gram négatif et non fermentantes.

Les documents a) « Development of a multispectral light-scatter sensor for bacterial colonies" de Huisung Kim et al., Journal of Biophotonics, 2016, b) "Hyperspectral imaging for presumptive identification of bacterial colonies on solid chromogenic culture media", de Guillemot Mathilde et al., proceedings of SPIE, 2016, c) "Hyperspectral microscope imaging methods to classifying gram-positive and gram-negative foodborne pathogenic bacteria" de Bossoon Park et al., Transaction of the American society of agricultural engineers, 2015, d) la demande de brevet européen EP 3 227 947, et e) "Hyperspectral fluorescence microscopy detects autofluorescent factors that can be exploited as a diagnostic method for species differentiation", de Graus Matthew et al., Journal of biomedical optics, 2017, décrivent des procédés de caractérisation de bactéries, leur identité ou leur gram, en analysant l'intensité lumineuse ou l'autofluorescence de colonies bactériennes. Ces documents sont cependant silencieux sur la possibilité de caractériser des levures.

Par réponse « électromagnétique naturelle », on entend au sens de l'invention que le microorganisme, une levure ou une souche bactérienne, n'est pas modifié à l'aide d'éléments (colorant, chromogène, fluorogène, etc.) qui modifient sa réponse électromagnétique à un éclairage au moins dans la gamme de longueurs d'onde d'intérêt. Par exemple, une colonie du microorganisme est cultivée dans un milieu nutritif non chromogène et non fluorescent et l'éclairage/acquisition se fait directement sur la colonie encore présente dans son milieu.

En d'autres termes, les inventeurs ont découvert que dans la gamme de longueurs d'onde 390nm-900nm les levures et les bactéries ont « naturellement » une signature électromagnétique permettant de les distinguer. Ainsi, il n'est pas nécessaire d'utiliser un substrat chromogène ou fluorogène ou des colorants. Par ailleurs, le procédé selon l'invention est rapide dans la mesure où il consiste à éclairer, mesurer un spectre et réaliser un traitement, notamment informatique, de ce spectre. Cette caractérisation du microorganisme au niveau levure ou bactérie permet par exemple d'optimiser un processus microbiologique de laboratoire tel que décrit précédemment.

On note que la caractérisation du microorganisme comme étant une levure ou une bactérie est réalisée directement à partir de l'intensité lumineuse acquise, sans nécessiter la détermination préalable de l'espèce du microorganisme. Notamment, le fait de ne pas avoir à identifier au niveau espèce présente l'avantage de grandement simplifier le modèle de prédiction puisque ce dernier peut être limité à deux classes.

Avantageusement, le procédé est appliqué à une boite de Pétri comprenant un milieu de nutritif gélosé sur lequel des colonies de microorganismes ont poussées. Par exemple, le milieu de nutritif est ensemencé à l'aide d'un échantillon biologique contenant, ou suspecté de contenir, des levures ou des bactéries, e.g. de l'urine, puis mis en culture pour faire pousser les colonies. Dès qu'une colonie est détectée sur le milieu nutritif, elle est caractérisée selon le procédé de l'invention. Ainsi, le procédé ne nécessite aucun transfert de matière ou d'ajout de réactif suite à l'ensemencement du milieu nutritif. La détection d'une colonie est par exemple réalisée de manière automatique par la prise à intervalle régulier d'images de la boite de Pétri et la mise en œuvre d'un algorithme de détection de colonies.

Avantageusement, le procédé selon l'invention ne se fonde pas sur l'analyse de l'autofluorescence du microorganisme mais sur l'analyse de sa réflectance ou de son absorbance. En particulier, l'éclairage est généralement trop intense pour que l'autofluorescence soit observable sur une image hyperspectrale ou multispectrale.

## EXPOSE DE L'INVENTION

Le but de la présente invention est de proposer un procédé de caractérisation d'un microorganisme qui soit automatique et qui ne nécessite pas de marquer ou de colorer le microorganisme ou son milieu de culture pour de déterminer ces caractéristiques.

A cet effet, l'invention est telle que définie aux revendications 1 et 23.

Selon un mode de réalisation, le procédé comporte en outre la détection du type de Gram et du caractère fermentant d'une souche bactérienne, comportant :

- l'éclairage dans la gamme de longueurs d'onde 390nm-900nm d'au moins une bactérie de ladite souche ayant une réponse électromagnétique naturelle dans ladite gamme;
- l'acquisition, dans ladite gamme, d'une intensité lumineuse réfléchie par, ou transmise au travers de, ladite bactérie éclairée; et
- la détermination du type de Gram et du caractère fermentant de la souche bactérienne en fonction de l'intensité lumineuse acquise dans ladite gamme

En d'autres termes, les bactéries ont « naturellement » une signature électromagnétique caractéristique de son type de Gram et de son caractère fermentant ou non. Le procédé selon l'invention consiste ainsi à mesurer cette signature puis à en extraire le type de Gram et le caractère fermentant de la bactérie. En particulier, grâce à l'invention il est possible de déterminer grâce à la gamme 390-900nm si la souche bactérienne est de Gram positif ou de Gram négatif et fermentante ou de Gram négatif et non fermentante, la connaissance de cette information permettant par exemple d'optimiser un processus microbiologique de laboratoire tel que décrit précédemment.

L'invention a également pour objet un procédé de calibration d'un système pour la mise en œuvre d'un procédé selon l'invention , le système comprenant :

- un éclairage configuré pour éclairer, dans la gamme de longueurs d'onde 390nm-900 nm, un microorganisme;
- un capteur configuré pour acquérir, dans la gamme 390nm-900nm, une intensité lumineuse réfléchie par, ou transmise au travers de, ledit microorganisme éclairée; et
- une unité informatique comprenant une mémoire informatique apte à contenir des instructions d'analyse de l'intensité acquise par le capteur et un microprocesseur apte à exécuter les instructions d'analyse contenues dans la mémoire informatique,

le procédé de calibration comprenant les étapes :

- de constitution d'une base de données d'apprentissage comprenant des intensités lumineuses dans la gamme 390nm-900nm de bactéries et de levures éclairées dans ladite gamme ;
- de mise en œuvre par ordinateur d'un apprentissage automatisé d'un modèle de prédiction de levure ou de bactérie en fonction de ladite base de données ; et
- de mémorisation, dans la mémoire informatique du système, d'instructions d'analyse pour la mise en œuvre du modèle de prédiction appris.

L'invention a également pour objet un procédé thérapeutique comprenant :

- le prélèvement d'un échantillon sur un patient suspecté d'être atteint d'une infection par une levure ou une bactérie ;
- la détection d'un ou de plusieurs microorganismes présents dans l'échantillon, avantageusement par l'ensemencement d'un milieu de culture gélosé avec l'échantillon, la mise en culture dudit milieu ensemencé pour faire pousser des colonies de microorganisme et la détection d'une ou plusieurs colonies ayant poussées ;
- la détermination du microorganisme comme étant une levure ou une souche bactérienne en fonction de l'intensité lumineuse acquise dans ladite gamme;
- le choix d'un ou de plusieurs antimicrobiens en fonction du résultat de ladite détermination ; et
- l'administration au patient du ou des antimicrobiens choisis.

## BREVE DESCRIPTION DES FIGURES

L'invention sera mieux comprise à la lecture de la description qui va suivre, donnée uniquement à titre d'exemple, et faire en relation avec les dessins annexés, dans lesquels des références identiques désignent des éléments identiques ou analogues, et dans lesquels

- la figure 1 est une vue schématique d'un système hyperspectral selon l'invention ;
- la figure 2 est une vue schématique d'un système multispectral selon l'invention ;
- la figure 3 est un exemple de spectre en transmission d'un filtre passe-bande utilisée dans le système de la figure 2 ;
- la figure 4 est un organigramme d'un procédé de prédiction des classes Y, GP, GNF, GNN mis en œuvre à l'aide du système de la figure 1 ou de la figure 2 ;
- la figure 5 est un organigramme d'un procédé de sélection de canaux spectraux discriminants à l'aide de l'approche « step forward » ;
- la figure 6 est un organigramme d'un procédé d'apprentissage des modèles de prédiction des classes Y, GP, GNF, GNN ;

- la figure 7A est un graphique illustrant un modèle de prédiction à plat des classes Y, GP, GNF, GNN ;
- la figure 7B est un graphique illustrant un modèle de prédiction hiérarchisé selon un arbre phylogénique des classes Y, GP, GNF, GNN ;
- la figure 7C est un graphique illustrant un modèle de prédiction hiérarchisé selon un arbre optimal des classes Y, GP, GNF, GNN ;
- la figure 8 est une table décrivant les espèces bactériennes et de levure utilisés pour l'apprentissage de modèle de prédiction des classes Y, GP, GNF, GNN ;
- les figures 9 et 10 sont des tables, respectivement pour le milieu COS et le milieu TSA, décrivant le nombre de pixels, et donc de spectres, utilisés pour l'étalonnage et la validation croisée des modèles de prédiction ;
- la figure 11 est un graphique illustrant le calcul d'un taux de prédiction pondéré, ou « balanced classification rate »
- la figure 12 est un graphique illustrant la répartition spatiale des principaux canaux spectraux discriminants pour l'arbre optimal du milieu COS ;
- la figure 13 est un graphique illustrant la répartition spatiale des 5 principaux canaux spectraux discriminants de chaque modèle pour l'arbre optimal du milieu COS ;
- la figure 14 est un graphique illustrant individuellement les cinq canaux discriminants principaux de chaque modèle de l'arbre optimal du milieu COS ;
- la figure 15 est un graphique illustrant la répartition spatiale des principaux canaux spectraux discriminants pour l'arbre optimal du milieu TSA ;
- la figure 16 est un graphique illustrant la répartition spatiale des principaux canaux spectraux discriminants pour l'arbre optimal du milieu COS
- la figure 17 est un graphique illustrant respectivement les 12, 8 et 11 canaux discriminant principaux associés respectivement aux premier, deuxième et troisième modèle de prédiction de l'arbre optimal du milieu TSA ;
- les figures 18 à 20 sont des graphiques illustrant respectivement les premier, deuxième et troisième modèles de prédiction de l'arbre phylogénique, le graphique en haut de chaque figure correspondant au milieu TSA et le graphique en bas de chaque figure correspondant au milieux COS.

## DESCRIPTION DETAILLEE DE L'INVENTION

Dans ce qui suit la notation $A_{i,j}$ se rapporte à l'élément de la i[ème] ligne et de la j[ième] colonne de la matrice A.

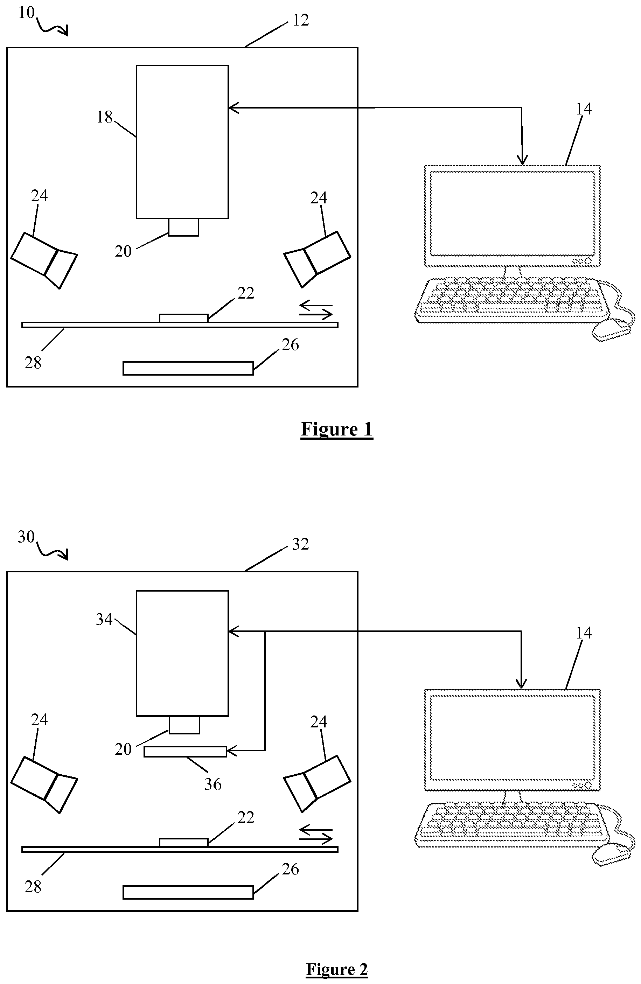

En se référant à la figure 1, un système hyperspectral **10** de caractérisation de colonies de levures ou de bactéries ayant poussées sur une gélose coulée dans une boite de Pétri comporte :

- un dispositif **12** d'acquisition d'image hyperspectrale ; et
- une unité **14** informatique de traitement de données connectée (e.g. par une liaison filaire ou sans fil), au dispositif **12** pour son pilotage et pour la réception et le traitement des images acquises par le dispositif **12.**

Le dispositif **12,** par exemple un système d'imagerie hyperspectrale de référence « Pika II » de la société Resonon, Montana USA, comporte :

- une caméra dite « hyperspectrale » **18,** constituée d'un capteur numérique comprenant un réseau de capteurs élémentaires, par exemple un capteur numérique de type CCD ou CMOS, sensible dans la gamme de longueurs d'onde $[\lambda_{min}; \lambda_{max}]$ = [390; 900] nanomètres, et d'un élément dispersif de lumière ou d'un spectrographe pour sélectionner une longueur d'onde à acquérir par le capteur ;
- un objectif **20** pour focaliser sur le capteur numérique de la caméra **18,** l'image optique d'une boite de Pétri **22,** dont on cherche à acquérir une image hyperspectrale ;
- un éclairage avant **24,** par exemple constitué d'une ou plusieurs lampes allogènes, e.g. 2 ou 4 lampes, apte à émettre de la lumière dans la gamme $[\lambda_{min}; \lambda_{max}]$ et pour réaliser un éclairage avant uniforme de la boite de Pétri **22.** Par exemple, les éclairages sont des lampes à lumière blanche ;
- un éclairage arrière **26,** par exemple constitué d'une matrice de LED à lumière blanche, pour réaliser un éclairage arrière uniforme de la boite de Pétri **22** dans la gamme $[\lambda_{min}; \lambda_{max}]$ ; et
- un chariot **28** sur lequel repose boite de Pétri **22** et permettant à cette dernière de défiler devant l'objectif **20** afin d'obtenir une image entière de la boite **22** par balayage.

Un éclairage est ainsi réalisé dans toute la gamme $[\lambda_{min}; \lambda_{max}]$.

Le dispositif **12** est par exemple configuré pour acquérir l'image d'une région de 90 millimètres par 90 millimètres avec un pas d'échantillonnage de 160 micromètres (résolution spatiale estimée à 300 micromètres) et avec une résolution spectrale de 1,7 nanomètre sur la gamme $[\lambda_{min}; \lambda_{max}]$.

Le dispositif **12** produit ainsi une image numérique *HSI* de la lumière réfléchie par la boite de Pétri, ayant N lignes et M colonnes, la boite de Pétri **22** étant préférablement ouverte (i.e. sans son couvercle):

$$HSI(\lambda) = \begin{pmatrix} Rad_{1,1}(\lambda) & ... & Rad_{1,j}(\lambda) & ... & Rad_{1,M}(\lambda) \\ \vdots & \ddots & \vdots & \vdots & \vdots \\ Rad_{i,1}(\lambda) & ... & Rad_{i,j}(\lambda) & ... & Rad_{i,M}(\lambda) \\ \vdots & \vdots & \vdots & \ddots & \vdots \\ Rad_{N,1}(\lambda) & ... & Rad_{N,j}(\lambda) & ... & Rad_{N,M}(\lambda) \end{pmatrix} \quad (1)$$

La radiance d'un pixel, communément appelée « intensité lumineuse », correspond ici à la quantité de lumière incidente sur la surface du site sensible élémentaire correspondant du capteur de la caméra **18** pendant la durée d'exposition, comme cela est connu en soi du domaine de la photographie numérique par exemple.

Chaque pixel $Rad_{i,j}(\lambda)$ se compose d'un spectre numérique de la radiance de la boite **22** correspondant au pixel à différentes longueurs d'onde $[\lambda_{min}; \lambda_{max}]$, le spectre numérique s'exprimant selon la relation :

$$\forall (i,j)\epsilon[1,N] \times [1,M]: Rad_{i,j}(\lambda) = \begin{pmatrix} Rad_{i,j}(\lambda_{min}) \\ Rad_{i,j}(\lambda_{min} + \Delta\lambda) \\ Rad_{i,j}(\lambda_{min} + 2 \times \Delta\lambda) \\ \vdots \\ Rad_{i,j}(\lambda_{min} + p \times \Delta\lambda) \\ \vdots \\ Rad_{i,j}(\lambda_{max}) \end{pmatrix} \quad (2)$$

où $\Delta\lambda$ est la résolution spectrale et $p$ est un entier positif appartenant à $\left[0, P = \frac{\lambda_{max} - \lambda_{min}}{\Delta\lambda}\right]$. Les longueurs d'onde d'acquisition $\lambda_{min}$ + $p \times \Delta\lambda$ sont usuellement désignées sous le termes de « canaux ».

L'unité **14** de traitement de données est par exemple un ordinateur personnel, une tablette, un smartphone, un serveur, un supercalculateur, ou plus généralement tout système à base de microprocesseur(s), notamment de type DSP (« digital signal processor »), à base de circuits de type FPGA, à base de circuits mixant ces types de technologie, etc., configuré pour mettre en œuvre un traitement des images *HSI* produites par le dispositif d'acquisition **12**. L'unité **14** est notamment pourvue de l'ensemble des mémoires (RAM, ROM, cache, mémoire de masse,...) pour la mémorisation des images produites par le dispositif **12**, d'instructions informatiques pour la mise en œuvre du procédé selon l'invention, de paramètres utiles à cette mise en œuvre et pour la mémorisation des résultats des calculs intermédiaires et finaux. L'unité **14** comporte optionnellement un écran d'affichage pour la visualisation du résultat final de la caractérisation des colonies, en particulier la détermination du type de Gram et/ou du caractère fermentant, et/ou du caractère bactérien ou de levure des colonies étudiées. Bien qu'une seule unité de traitement soit décrite, l'invention s'applique évidemment à un traitement réalisé par plusieurs unités de traitement (e.g. une unité embarquée dans la caméra **18** pour mettre en œuvre un prétraitement de images *HSI* et une unité externe au dispositif **12** pour la mise en œuvre du reste du traitement). Par ailleurs, le système peut être complété par une interface permettant d'entrer dans l'unité **14** des données relatives à l'échantillon, notamment le type de milieu de culture utilisé lorsque la prédiction dépend du milieu, par exemple au moyen d'un clavier/souris et d'un menu déroulant à disposition de l'opérateur, un lecteur de code barre/QR code lisant un code barre/QR code présent sur la boite de Pétri et comprenant des informations sur le milieu, etc.

Le système hyperspectral de la figure 1 a pour avantage d'être agile en termes de longueurs d'onde d'acquisition car il peut s'adapter à différents modèles de prédiction de la classe des colonies et utiliser un nombre important de canaux spectraux pour augmenter la précision de la prédiction. Outre un prix élevé, un tel système est toutefois généralement moins résolu spatialement qu'une caméra CMOS ou CCD classique dont le but est uniquement d'acquérir une image en intensité de la lumière incidente sur son capteur.

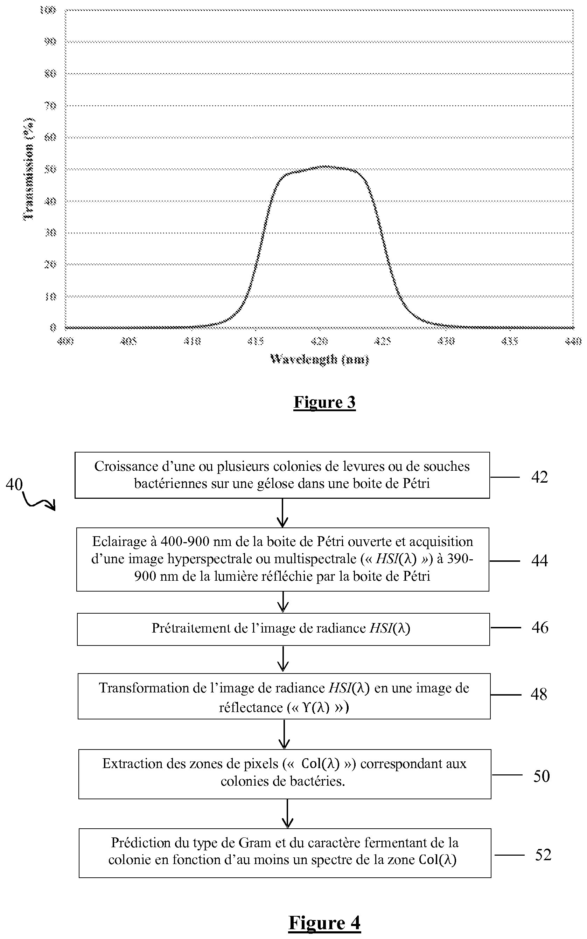

En se référant à la figure 2, un système multispectral **32** diffère du système hyperspectral **10** par la caméra **32**, avantageusement une caméra CMOS ou CCD de haute résolution spatiale, couplée à un ensemble de filtres spectraux **36**, par exemple disposé devant l'objectif **20** entre l'objectif **20** et le capteur de la caméra **32**. L'ensemble de filtres **36** est constitué d'un nombre $N_F$ de filtres passe-bandes distincts, chacun configuré pour transmettre uniquement la lumière dans une gamme $[\lambda_1; \lambda_2]$ de la gamme $[\lambda_{min}; \lambda_{max}]$, avec une largeur spectrale à mi-hauteur du maximum (ou FWHM pour « full width half maximum ») inférieure ou égale à 50nm, et de préférence inférieure ou égale à 20nm. Le spectre en transmission d'un tel filtre, e.g. un filtre de la société Edmund Optics centrée sur 420nm, est illustré à la figure 3.

L'ensemble **36** est par exemple une roue à filtres pouvant accueillir jusqu'à 24 filtres différents, roue pilotée par l'unité **14** qui l'actionne pour faire défiler devant la caméra lesdits filtres et commander une prise d'image pour chacun d'entre eux.

Il est ainsi acquis une image multispectrale $HSI(\lambda)$ dont chaque pixel $Rad_{i,j}(\lambda)$ se compose d'un spectre numérique de la radiance de la boite **22** correspondant au pixel aux différentes bandes spectrales filtrées par l'ensemble **36,** le spectre numérique s'exprimant selon la relation :

$$\forall (i,j) \epsilon [1,N] \times [1,M]: Rad_{i,j}(\lambda) = \begin{pmatrix} Rad_{i,j}(\lambda_1) \\ Rad_{i,j}(\lambda_2) \\ \vdots \\ Rad_{i,j}(\lambda_{N_F}) \end{pmatrix} \tag{3}$$

Où $\lambda_1, \lambda_2, ..., \lambda_{N_F}$ sont respectivement les longueurs d'onde centrales des filtres spectraux de l'ensemble **36.**

Un procédé **40** de caractérisation de microorganismes contenus dans un échantillon biologique (e.g. urine, sang, prélèvement broncho-alvéolaire, etc.) au moyen du système venant d'être décrit est à présent détaillé en relation avec l'organigramme de la figure 4. En particulier, ce procédé trouve avantageusement application dans le cadre d'un procédé plus global d'identification des microorganismes contenus dans ledit échantillon à l'aide d'une spectrométrie de masse MALDI-TOF (e.g. Vitek® MS commercialisée par la Demanderesse) et d'un antibiogramme desdits microorganismes (e.g. à l'aide de la plateforme Vitek® 2 commercialisée par la Demanderesse). Comme cela est connu en soi, chacune de ces techniques nécessite de choisir des milieux et/ou des réactifs et/ou des consommables particuliers en fonction du type de microorganisme. A titre d'exemple, l'identification de levures par spectrométrie de masse MALDI-TOF implique avantageusement l'emploi d'acide formique dans la matrice utilisée dans ce type de technologie. De même, le choix de la carte de la plateforme Vitek® 2 (carte comprenant un milieu de pousse et un ou plusieurs antimicrobiens testés lors de l'antibiogramme) dépend du caractère bactérien du microorganisme testé. Notamment, les bactéries à Gram négatif et fermentantes nécessitent une carte particulière pour réaliser leur antibiogramme.

De manière avantageuse, le procédé de caractérisation **40** de caractérisation décrit ci-après permet, dès la première pousse sur boite de Pétri, d'obtenir les informations nécessaires sur les microorganismes pour la suite du processus microbiologique, en particulier de savoir si une colonie ayant poussé correspond à une levure (« Y ») ou une bactérie, et dans le cas d'une bactérie, si cette bactérie est de type Gram positif (« GP ») ou de type Gram négatif (« GN »), et dans le cadre de bactérie de type Gram négatif, si cette bactérie est fermentante (« GNF ») ou non fermentante (« GNN »). Le procédé permet ainsi de prédire la classe d'un microorganisme, à s'avoir la classe Y, GP, GNF ou GNN.

Dans une première étape **42** du procédé, une boite de Petri est ensemencée par un échantillon biologique, e.g. prélevé sur un patient, de sorte à faire pousser des colonies de levure ou de bactérie à la surface d'un milieu nutritif, ou « de culture », déposé dans la boîte de Pétri. Le milieu nutritif, a pour but principal de faire pousser ladite colonie, et optionnellement de renforcer la précision de la caractérisation en limitant les perturbations lumineuses. De préférence concernant une détection du type de Gram en fonction de l'intensité lumineuse réfléchie, le milieu nutritif est opaque, ce qui augmente le degré de précision de la détection. Notamment, le milieu opaque a un facteur de réflectance $\rho$ inférieur ou égal à 10%, et de préférence inférieur ou égal à 5%, et de manière encore plus préférentielle inférieur ou égal à 1%. Par exemple, le milieu de culture une gélose dite « CPSO » (gélose « CPS » comprenant du $SiO_2$ pour opacifier le milieu), une gélose dite « columbia » (ou gélose « CNA »), une gélose Columbia avec 5% de sang de mouton (ou gélose dite « COS »), une gélose de Man, Rogosa, Sharpe (gélose dite « MRSM »), une gélose chocolat (gélose dite « PVX »), une gélose Tryptone-Soja (gélose dite « TSA »), etc.

Ce type de pousse de colonies étant classique, il ne sera pas décrit plus en détail par la suite. Il peut avantageusement être mis en œuvre manuellement par un opérateur ou automatiquement à l'aide d'un automate d'ensemencement d'une manière connue en soi. De manière avantageuse, la préparation est réalisée de sorte que les colonies, sur la base desquels la caractérisation du microorganisme est réalisée, sont distancées les unes des autres et de sorte que la surface d'une colonie corresponde à une pluralité de pixels dans l'image acquise par le dispositif **12.** Ceci permet notamment de faciliter leur identification ultérieure dans l'image acquise, et donc leur segmentation au moyen d'une algorithme de traitement d'image ou leur extraction dans l'image par un utilisateur.

Une fois la pousse des colonies terminée, par exemple après une durée de 24h, 36h ou 48h, la boite de Pétri est de préférence ouverte, disposée sur le chariot **28,** les éclairages **24** et **26** sont allumés et au moins une image hyperspectrale (respectivement multispectrale) $HSI$ de la boite de Pétri est acquise, en **44,** à l'aide du dispositif d'acquisition **12** (respectivement **32**) et mémorisée dans l'unité de traitement **14,** qui met en œuvre un traitement informatique pour déterminer le type microorganisme constitutif de la colonie à partir des images acquises.

L'unité **14** débute optionnellement, en **46,** par un prétraitement du bruit, consistant en l'un des traitements suivants ou une combinaison quelconque de ces traitements :

a. une correction du bruit du capteur de la caméra, notamment son offset, son bruit spatial, etc., d'une manière connu en soi ;

b. un traitement des réflexions parasites, notamment spéculaire formant des « surbrillances » dans l'image *HSI*. Par exemple, un seuillage mis en œuvre pour éliminer les pixels ayant des valeurs supérieures à un seuil prédéterminé, e.g. supérieures ou égales aux deux tiers de la valeur maximale que peuvent prendre les pixels (i.e. supérieures ou égales à 170 dans le cas de pixels codés sur 8 bits entre 0 et 255) ;

c. une traitement ratiométrique permettant d'atténuer les variations dans les images provoquées par des fluctuations extérieures telles que de variations de l'illumination, en divisant l'image *HSI* par une intensité lumineuse réfléchie à une longueur d'onde qui est invariante avec le type de bactérie et le type de gélose utilisé ;

d. si plusieurs images *HSI* ont été acquises, la recherche et l'élimination de valeurs de pixels aberrantes et/ou la moyenne des images acquises.

De manière avantageuse, le traitement se poursuit, en **48,** par la transformation de l'image *HSI* prétraitée, qui mémorise des valeurs de radiance à différentes longueurs d'onde, en une image hyperspectrale ou multispectrale de réflectance afin d'extraire le signal généré par la boite de Pétri seule. Ceci permet notamment de filtrer les fluctuations du spectre d'émission des sources d'éclairage **24, 26.** Par exemple, une correction du type « *flat field correction* » (FFC) est mise en œuvre pour obtenir la réflectance, ce qui présente en outre l'avantage de corriger les dispersions de réponse du capteur de pixel à pixel (dispersion du courant d'obscurité, dispersion du gain, etc.).

Dans le cadre d'une image hyperspectrale, cette transformation est par exemple une correction selon les relations :

$$\forall (i,j) \epsilon [1,N] \times [1,M], \forall p \in [0,P]:$$

$$\Upsilon_{i,j}(\lambda_{min} + p \times \Delta\lambda) = \frac{Rad_{i,j}(\lambda_{min} + p \times \Delta\lambda) - B_{i,j}(\lambda_{min} + p \times \Delta\lambda)}{W_{i,j}(\lambda_{min} + p \times \Delta\lambda) - B_{i,j}(\lambda_{min} + p \times \Delta\lambda)} \times m(\lambda_{min} + p \times \Delta\lambda) \qquad (4)$$

où $\Upsilon(\lambda)$ est une image en réflectance, W est une image hyperspectrale mémorisée dans l'unité **14** d'un objet neutre de réflectance élevée et illuminé par les éclairages **24, 26,** par exemple une feuille de réflectance uniforme supérieure à 90% (e.g. une feuille dite « blanche » ou à une charte de gris inférieur à 10%), et *B* est une image hyperspectrale mémorisée dans l'unité **14** d'un objet neutre de réflectance faible, par exemple l'image d'un capuchon noir obturant l'objectif **20** et *m* ($\lambda_{min} + p \times \Delta\lambda$) = 1 ou égal à la moyenne de la matrice $W(\lambda_{min} + p \times \Delta\lambda)$ - $B(\lambda_{min} + p \times \Delta\lambda)$.

De manière analogue, dans le cadre d'une image multispectrale, la transformation est par exemple une correction selon la relation :

$$\forall (i,j) \epsilon [1,N] \times [1,M], \forall n \in [1,N_f]:$$

$$\Upsilon_{i,j}(\lambda_n) = \frac{Rad_{i,j}(\lambda_n) - B_{i,j}(\lambda_n)}{W_{i,j}(\lambda_n) - B_{i,j}(\lambda_n)} \times m(\lambda_n) \qquad (5)$$

où *W* est une image multispectrale mémorisée dans l'unité **14** d'un objet neutre de réflectance élevée et illuminé par les éclairages **24, 26,** par exemple une feuille de réflectance uniforme supérieure à 90% (e.g. une feuille dite « blanche » ou à une charte de gris inférieur à 10%), et *B* est une image multispectrale mémorisée dans l'unité **14** d'un objet neutre de réflectance faible, par exemple l'image d'un capuchon noir obturant l'objectif **20** et $m(\lambda_n)$ = 1 ou égal à la moyenne de la matrice $W(\lambda_n)$ - $B(\lambda_n)$.

L'unité **14** met en œuvre en **50,** suite à l'étape **38** ou en parallèle des étapes précédentes, un algorithme d'identification des colonies de bactéries, e.g. à partir de l'image $HSI(\lambda)$ ou $\Upsilon(\lambda)$. Tout algorithme de reconnaissance de forme et d'objet classique peut être mis en œuvre pour extraire une zone de l'image, nommée « Col($\lambda$) », correspondant à une colonie. En variante, cette sélection est faite manuellement par un opérateur qui sélectionne cette zone en s'aidant de l'écran d'affichage et d'un mécanisme de pointage du type souris par exemple. A titre d'exemple, la zone Col($\lambda$) consiste en une liste des coordonnées de pixel appartenant à la colonie. Les zones de pixels sélectionnées sont mémorisées par l'unité **14.**

Le procédé se poursuit, en **52,** par la prédiction de la classe Y, GP, GNF ou GNN du microorganisme de la colonie en fonction d'au moins un spectre de la zone Col($\lambda$) en appliquant des règles de décision prédéfinies, dont des variantes sont décrites ci-après. En particulier, cette prédiction est réalisée en fonction du spectre $\Upsilon_{i,j}(\lambda)$ de chaque pixel $(i,j)$ de la zone Col($\lambda$). A cet effet, une première prédiction de la classe est réalisée pour chaque pixel $(i,j)$ de la zone Col($\lambda$), puis un vote majoritaire est mis en œuvre pour la prédiction finale de la classe. Dans une première variante, un vote majoritaire simple est mise en œuvre, c'est-à-dire que la classe prédite sur le plus grand nombre de pixels de la zone Col($\lambda$) est la classe finalement retenue. Dans une seconde variante, afin d'augmenter la certitude dans la prédiction de la classe, un vote qualifié est mis en œuvre, c'est-à-dire que la classe finalement retenue est celle qui est prédite sur plus de $X$% du nombre de pixels constitutifs de la zone Col($\lambda$), avec $X$ supérieur strictement à 50%, et de préférence supérieur ou égal à 70%. Si aucune classe ne remplit cette condition, le procédé renvoie alors une absence de prédiction de classe. Bien entendu, la classe de la colonie peut être réalisée à l'aide d'une seule valeur, par exemple le spectre moyen $\Upsilon_{col}(\lambda)$ de l'ensemble $\{\Upsilon_{i,j}(\lambda)\}_{(i,j)\in Col(\lambda)}$ des spectres de la zone Col($\lambda$).

La classe Y, GP, GNF ou GNN de chaque colonie par l'unité **14** est réalisée en appliquant des règles de prédiction prédéfinies, dont des variantes sont décrites ci-après. Les classes prédites sont mémorisées dans l'unité **14** et/ou affichées sur un écran à l'attention de l'utilisateur. Cette prédiction est également avantageusement délivrée à un autre instrument d'analyse microbienne pour une étape ultérieure d'identification et/ou d'antibiogramme des microorganismes ayant formé les colonies.

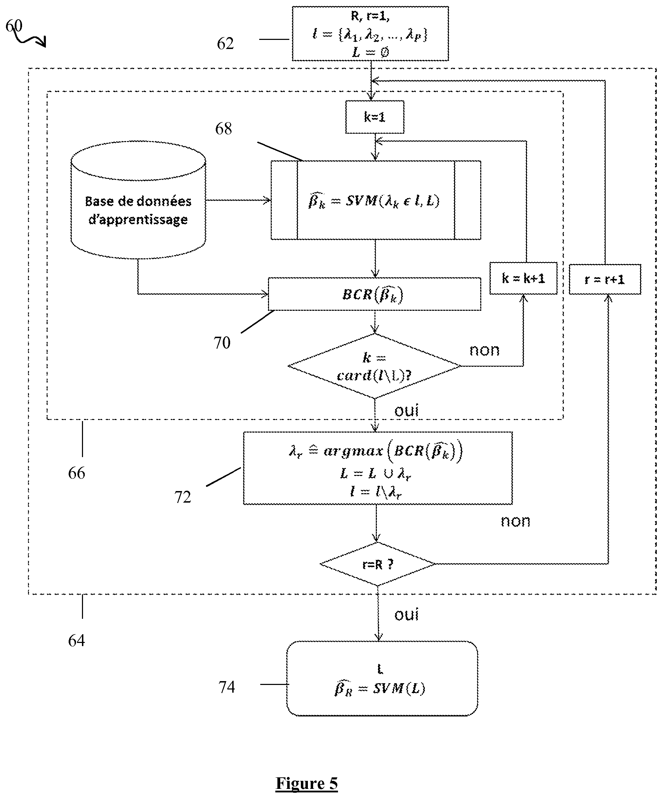

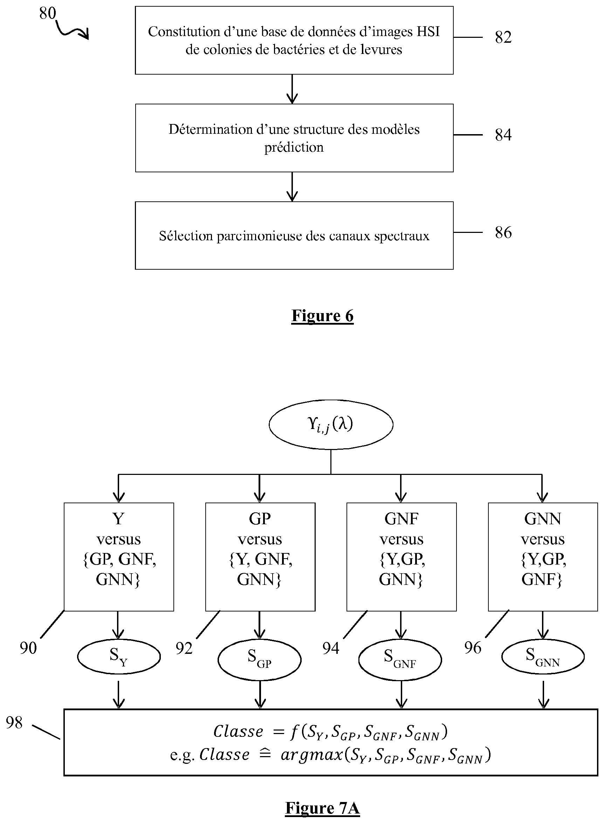

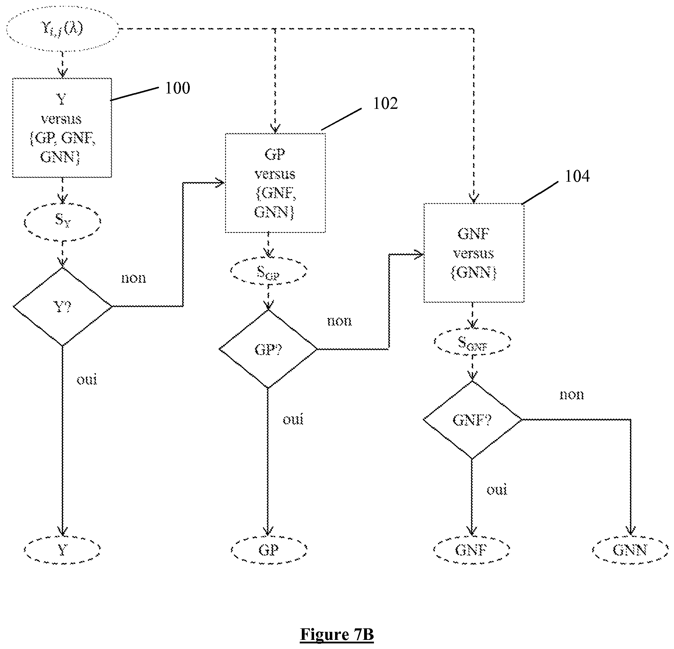

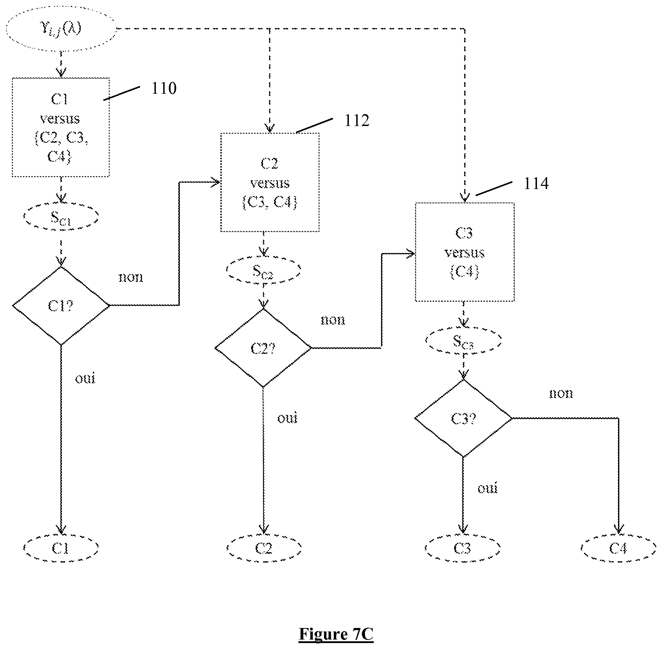

Il va à présent être décrit différents modèles de prédiction d'une classe Y, GP, GNF ou GNN en fonction d'un spectre $\Upsilon_{i,j}(\lambda)$ d'un pixel d'une colonie, notamment des modèles de prédiction basés sur un apprentissage automatique supervisé (« SML », pour « supervised machine learning »). Les outils SML utilisés sont tout d'abord décrits en relation avec la figure 5 puis les modèles de prédiction sont décrits ci-dessous au travers de leur procédé d'apprentissage illustré aux figures 6 et 7.

A. OUTILS D'APPRENTISSAGE AUTOMATIQUE SUPERVISE

Quel que soit l'apprentissage considéré, celui-ci débute par la constitution d'une base de données d'apprentissage. Pour chaque classe Y, GP, GNF et GNN, des bactéries et des levures sont sélectionnées et chacune d'entre elle est ensemencée sur une gélose coulée dans une boite de Pétri, mise en culture pendant une durée prédéterminée et une image hyperspectrale de la boite est acquise avec le système décrit à la figure 1, et par conséquent sous les mêmes conditions d'illumination et dans gamme de longueurs d'onde 390nm-900nm. Les pixels des colonies ayant poussé sur la gélose sont extraits, par exemple de la manière décrite aux étapes **46-50** du procédé **40,** et leurs spectres associés mémorisés dans la base de données d'apprentissage. Cette dernière comprend ainsi quatre ensemble de spectres $\{\Upsilon_m^Y(\lambda)\}$, $\{\Upsilon_m^{GP}(\lambda)\}$, $\{\Upsilon_m^{GNF}(\lambda)\}$, $\{\Upsilon_m^{GNN}(\lambda)\}$ respectivement associés aux classes Y, GP, GNF, GNN. Chacun de ces ensembles est scindé en deux, une première partie, dite « d'étalonnage » étant utilisé pour l'apprentissage lui-même et une deuxième partie, dite « de validation croisée », étant utilisée pour d'évaluer la performance des modèles de prédiction calculés, comme cela est connu en soi de l'état de la technique.

Selon un premier mode de réalisation privilégié, un apprentissage mis en œuvre par ordinateur, dit « step forward », est mis en œuvre pour l'apprentissage des modèles de prédiction. Ce type d'apprentissage repose sur la sélection pas à pas des canaux spectraux les plus discriminants, de sorte qu'il est intrinsèquement parcimonieux et adapté à la recherche d'une application multispectrale telle que mise en œuvre par le système de la figure 2.

En se référant à la figure 5, l'apprentissage **60** débute par une étape d'initialisation **62** dans laquelle un nombre maximal $R$ de canaux discriminants est sélectionné, ce nombre étant compris entre 1 et le nombre $P$ de canaux spectraux de la caméra hyperspectrale utilisée pour l'acquisition des spectres. Une liste L des canaux discriminants sélectionnés est vidée et une liste $I$ des canaux candidats est initialisée à l'ensemble $\{\lambda_1, \lambda_2, ..., \lambda_P\}$ des canaux de la caméra hypespectrale.

Dans une étape itérative **64** suivante, la liste $I$ est remplie pas à pas avec les R canaux les plus discriminants de la liste $L$ en mettant en œuvre une étape itérative **66.** Plus particulièrement pour une itération $r$ donnée de l'étape **64,** l'étape **66** :

- extrait chaque canal $\lambda_k$ de la liste $I$ ;

- détermine, en **68,** pour le canal extrait $\lambda_k$ et les canaux $\{\lambda_1, \lambda_2, ..., \lambda_{r-1}\}$ de la liste $L$, un modèle de prédiction $\widehat{\beta_k}$ ;

- calcule, en **70,** un critère de performance $BCR(\widehat{\beta_k})$ du modèle de prédiction $\widehat{\beta_k}$, par exemple le taux de bonne classification des spectres de validation croisée.

L'étape **64** se poursuit alors, en **72,** par l'identification du modèle de prédiction donnant le meilleurs critère de performance et par conséquent l'identification du canal $\lambda_r$ de la liste $I$ le plus discriminant en combinaison avec les canaux de la liste $L$. En **74,** la liste $L$ est alors complétée avec le canal $\lambda_r$ et ce dernier est retiré de la liste $I$ pour l'itération $r+1$ suivante de l'étape **64.** Une fois les $R$ canaux les plus discriminants identifiés, le procédé d'apprentissage se termine alors, en **74**, par la mémorisation de la liste *L* et de l'hyperplan prédiction $\left(\widehat{\beta_R}, \beta_0^{cl}\right)$ associé à cette dernière, à savoir le dernier modèle identifié lors de l'étape **72**.

De manière avantageuse, les modèles de prédiction calculés à l'étape **68** sont de type SVM (pour « support vector machine »), « un contre tous », à noyau linéaire (ou « linear kernel ») et à marge souple (ou « soft margin »). Ce type d'apprentissage consiste à calculer, en fonction des spectres d'étalonnage, un hyperplan $\left(\widehat{\beta_k^{Cl}}, \beta_0^{cl}\right)$ séparant une classe *Cl* (*Cl* = Y, GP, GNF ou GNN) de l'ensemble $\overline{Cl}$ formé d'une, deux ou trois parmi les autres classes, comme cela sera décrit ci-après. Par exemple, le modèle est appris en résolvant un problème d'optimisation selon les relations suivantes pour une itération *k* de l'étape **66** et une itération *r* de l'étape **64** :

$$\widehat{\beta_k^{Cl}} = \arg \min_{\beta, \xi_m} \left( \frac{1}{2} \|\beta\|^2 + C \sum_{m=1}^{M} \xi_m \right)$$

sous les contraintes :

$$\forall m \in [1, M] : \xi_m \geq 0 \qquad\qquad (6)$$

$$\forall m \in [1, M] : q_m \left( \Upsilon_m^{r,k}(\lambda).\beta + \beta_0^{cl} \right) \geq 1 - \xi_m$$

expressions dans lesquelles :

- pour un spectre d'étalonnage $\Upsilon_m(\lambda)$ appartenant à la classe *Cl ou* à l'ensemble $\overline{Cl}$, $\Upsilon_m^{r,k}(\lambda)$ est égal au vecteur des composantes de $\Upsilon_m(\lambda)$ correspondant aux canaux spectraux de la liste $L = \{\lambda_1, \lambda_2, ..., \lambda_{r-1}\}$ et du canal $\lambda_k$ extrait de la liste *l* au cours de l'itération *k*, de préférence un vecteur dont les composantes sont ordonnées en fonction de la valeur des canaux ;

- $\widehat{\beta_k^{Cl}}$ et $\beta$ sont des vecteurs de dimension égale à la dimension des spectres $\Upsilon_m^{r,k}(\lambda)$, et par conséquent de dimension égale à *r*,

- *M* est le nombre de spectres d'étalonnage $\Upsilon_m(\lambda)$ appartenant à la classe *Cl ou* à l'ensemble $\overline{Cl}$, numérotés de 1 à M,

- $\Upsilon_m^{r,k}(\lambda).\beta$ est le produit scalaire entre le vecteur $\Upsilon_m^{r,k}(\lambda)$ et le vecteur $\beta$,

- $\xi_m$ et $\beta_0^{cl}$ sont des scalaires ;

- $q_m \in \{-1, 1\}$ avec $q_m = 1$ si le $m^{\text{ième}}$ spectre d'apprentissage est associé à la classe *Cl,* et $q_m = -1$ si le $m^{\text{ième}}$ spectre est associé à l'ensemble $\overline{Cl}$ des autres classes ; et

- *C* est un scalaire prédéfini.

Le modèle prédisant l'appartenance à la classe *Cl* d'un spectre d'un pixel $\Upsilon_{i,j}(\lambda)$ est ainsi réalisé selon les étapes suivantes :

- la transformation du spectre $\Upsilon_{i,j}(\lambda)$ en un vecteur $\Upsilon_{i,j}^{r,k}(\lambda)$ ;

- le calcul d'une distance $S_{cl} = \Upsilon_{i,j}^{r,k}(\lambda).\widehat{\beta_k} + \beta_0$ entre le spectre $\Upsilon_{i,j}^{r,k}(\lambda)$ et l'hyperplan $\left(\widehat{\beta_k^{Cl}}, \beta_0^{cl}\right)$ ;

- l'application d'une règle de prédiction de la classe *Cl* en fonction de la distance $S_{cl}$, par exemple le spectre appartient à la classe *Cl* si le signe de $S_{cl}$ est positif, et à l'ensemble $\overline{Cl}$ si ce signe est négatif.

Selon un deuxième mode de réalisation, l'apprentissage est non parcimonieux et consiste à utiliser tous les canaux en même temps, le modèle de prédiction en découlant étant particulièrement adapté à une application hyperspectrale à l'aide du système de la figure 1. Par exemple, cet apprentissage est de type SVM, « un contre tous », à noyau linéaire et à marge souple, et consiste à calculer, en fonction des spectres d'étalonnage, un hyperplan $\left(\widehat{\beta^{Cl}}, \beta_0^{cl}\right)$

séparant une classe *Cl* (*Cl* = Y, GP, GNF ou GNN) de l'ensemble $\overline{Cl}$ formé d'une, deux ou trois parmi les autres classes, en résolvant un problème d'optimisation selon la relation :

$$\widehat{\beta^{Cl}} = \arg \min_{\beta, \xi_m} \left( \frac{1}{2} \|\beta\|^2 + C \sum_{m=1}^{M} \xi_m \right)$$

sous les contraintes :

$$\forall m \in [1, M]: \xi_m \geq 0 \qquad\qquad (7)$$

$$\forall m \in [1, M]: q_m\big(\Upsilon_m(\lambda).\beta + \beta_0^{cl}\big) \geq 1 - \xi_m$$

expressions dans lesquelles :

- $\Upsilon_m(\lambda)$ est un spectre d'étalonnage $\Upsilon_m(\lambda)$ appartenant à la classe *Cl ou* à l'ensemble $\overline{Cl}$ ;
- $\widehat{\beta^{Cl}}$ et $\beta$ sont des vecteurs de dimension égale à la dimension des spectres d'étalonnage $\Upsilon_m^{r,k}(\lambda)$, et par conséquent de dimension égale à *P*,
- *M* est le nombre de spectres d'étalonnage $\Upsilon_m(\lambda)$ appartenant à la classe *Cl ou* à l'ensemble $\overline{Cl}$, numérotés de 1 à M,
- $\Upsilon_m(\lambda). \beta$ est le produit scalaire entre le vecteur $\Upsilon_m(\lambda)$ et le vecteur $\beta$,
- $\xi_m$ et $\beta_0^{cl}$ sont des scalaires ;
- $q_m \in \{-1, 1\}$ avec $q_m = 1$ si le *m*ième spectre d'apprentissage est associé à la classe *Cl,* et $q_m = -1$ si le *m*ième spectre est associé à l'ensemble $\overline{Cl}$ des autres classes ; et
- *C* est un scalaire prédéfini.

Le modèle prédisant l'appartenance à la classe *Cl* d'un spectre d'un pixel $\Upsilon_{i,j}(\lambda)$ est ainsi réalisé selon les étapes suivantes :

- le calcul d'une distance $S_{cl} = \Upsilon_{i,j}(\lambda). \widehat{\beta^{Cl}} + \beta_0^{cl}$ entre le spectre $\Upsilon_{i,j}(\lambda)$ et l'hyperplan $\left(\widehat{\beta^{Cl}}, \beta_0^{cl}\right)$ ;
- l'application d'une règle de prédiction de la classe *Cl* en fonction de la distance $S_{cl}$, par exemple le spectre appartient à la classe *Cl* si le signe de $S_{cl}$ est positif, et à l'ensemble $\overline{Cl}$ si ce signe est négatif.

## B. PROCEDE D'APPRENTISSAGE DES MODELES DE PREDICTION

En se référant à la figure 6, le procédé **80** d'apprentissage des modèles de prédiction débute par la constitution, en **82,** d'une base de données d'apprentissage pour les classes Y, GP, GNF et GNN, tel que décrit précédemment. Le procédé **80** se poursuit, en **84,** par la détermination d'une structure des modèles de prédiction. En particulier, deux types de modèle sont possibles, tels que respectivement illustrés à la figure 7A d'une part et aux figures 6B et 6C d'autre part.

Le premier type de modèle de prédiction, illustré à la figure 7A, consiste à apprendre quatre modèles de prédictions de type « un contre tous » **90, 72, 74, 76,** à savoir une prédiction de la classe Y contre les classes GP, GNF, GNN, une prédiction de la classe GP contre les classes Y, GNF, GNN, une prédiction de la classe GNF contre les classes Y, GP, GNN et une prédiction de la classe GNN contre les classes Y, GP, GNF. A cette fin :

- chaque modèle de prédiction est appris en fonction de la base de données d'apprentissage en mettant en œuvre un des outils d'apprentissage décrits ci-dessus en relation avec les relations (6) ou (7), la classe *Cl* étant égale à Y, GP, GNF GNN et l'ensemble $\overline{Cl}$ étant constitué des trois autres classes;
- La prédiction du type de Gram et du caractère fermentant d'un pixel (étape **52** de la figure 3) est obtenue en calculant (étapes **90-96**), les distance $S_Y$, $S_{GP}$, $S_{GNF}$, $S_{GNN}$ du spectre $\Upsilon_{i,j}(\lambda)$ dudit pixel respectivement aux hyperplans $\left(\widehat{\beta_R^Y}, \beta_0^Y\right), \left(\widehat{\beta_R^{GP}}, \beta_0^{GP}\right), \left(\widehat{\beta_R^{GNF}}, \beta_0^{GNF}\right), \left(\widehat{\beta_R^{GNN}}, \beta_0^{GNN}\right)$, ou aux hyperplans hyperplans $\left(\widehat{\beta^Y}, \beta_0^Y\right), \left(\widehat{\beta^{GP}}, \beta_0^{GP}\right), \left(\widehat{\beta^{GNF}}, \beta_0^{GNF}\right), \left(\widehat{\beta^{GNN}}, \beta_0^{GNN}\right)$, puis en déterminant la classe du pixel, en **88,** en fonction des distances calculées. Notamment, la classe retenue est celle correspondant à la distance maximale.

Selon le premier type de structure illustré à la figure 7A, dite « à plat », les classes Y, GP, GNF et GNN sont considérées d'égale importance, et par conséquent les erreurs d'identifications également. Par exemple, identifier une levure Y en lieu et place d'une bactérie GNN est aussi grave qu'identifier une bactérie GNF en lieu et place d'une bactérie GNN. Selon la structure illustrée à la figure 7B, les modèles de prédiction sont organisés selon un arbre taxonomique phylogénique, ce qui permet de ne plus considérer les différentes classes avec une égale importance et d'introduire une information a priori, à savoir une information d'évolution pouvant influencer la forme des spectres. Plus particulièrement, cet arbre de modèle de prédiction comporte :

-    un premier modèle **100** consistant à distinguer les levures Y des bactéries GP, GNF, GNF;
-    un deuxième modèle **102** consistant à distinguer les bactéries GP des bactéries GNF et GNN ; et
-    un troisième modèle **104** consistant à distinguer les bactéries GNF des bactéries GNN.

Chacun des modèles **100-104** est obtenu de la manière décrite précédemment en relation avec les relations (6) ou (7) et la prédiction de l'appartenance d'un spectre de pixel $\Upsilon_{i,j}(\lambda)$ à l'une des classes Y, GP, GNF et GNN consiste ainsi à calculer sa distance $S_Y$ à l'hyperplan du premier modèle **100** et si le signe de cette distance est positif, la classe Y est alors prédite. Sinon, la distance $S_{GP}$ à l'hyperplan du second modèle **102** est calculé et si le signe de celle-ci est positif, alors la classe GP est prédite. Sinon, la distance $S_{GNF}$ à l'hyperplan du troisième modèle **104** est calculé alors la classe GNF est prédite. Sinon la classe GNN est prédite.

Si le modèle phénotypique permet d'améliorer la précision de la prédiction par rapport à un structure de prédiction à plat telle qu'illustrée à la figure 7A, les inventeurs ont cependant noté que l'arbre phénotypique n'est pas nécessairement l'arbre donnant de meilleurs résultats. Notamment, un arbre peut être privilégié en fonction du milieu de culture sur lequel les microorganismes à caractériser ont poussé, milieu qui influence la forme des spectres. De préférence, une structure de prédiction optimale, telle qu'illustrée à la figure 7C, est déterminée en fonction des spectres d'étalonnage. Plus particulièrement, dans une première étape, les quatre modèles de prédiction a) Y contre GP, GNF et GNN, b) GP contre Y, GNF et GNN, c) GNF contre Y, GP et GNN, et d) GNN contre Y, GP et GNF sont calculés de la manière décrite ci-dessus et le modèle ayant la meilleure performance de prédiction est conservé pour être le premier modèle **110** de l'arbre optimal. Dans une seconde étape, la classe du premier modèle est écarté, et les trois modèles de prédiction correspondant aux classes restantes sont calculés. Par exemple, si le premier modèle correspond à la classe GNN, alors les trois modèles de la seconde étape sont a) Y contre GP et GNF, b) GP contre Y, et GNF et c) GNF contre Y et GP de la manière décrite ci-dessus. Le meilleur des trois modèles est alors conservé pour être le second modèle **112** de l'arbre optimal. Dans une troisième étape, les classes des premier et deuxième modèles **110** et **112** sont écartés, et un modèle de prédiction entre les deux classes restantes est calculé de la manière décrite ci-dessus et conservé en tant que troisième modèle **114** de l'arbre optimal. La prédiction de l'appartenance d'un spectre de pixel $\Upsilon_{i,j}(\lambda)$ à l'une des classes Y, GP, GNF et GNN est alors obtenue en parcourant l'arbre d'une manière analogue à celle décrite en relation avec la figure 7B.

En revenant à la figure 6, une fois la structure du modèle de prédiction déterminée, le procédé d'apprentissage **80** se poursuit, optionnellement, en **84,** par une diminution du nombre de canaux spectraux utilisés pour la prédiction, cette diminution étant réalisée par sélection et/ou par regroupement de canaux. En particulier, lorsque les modèles de prédiction précédemment décrits en relation avec les figures 7A, 7B et 7C, sont calculés grâce à l'approche « step forward » de la figure 5, le nombre de canaux utilisés peut être fixé directement par le paramètre *R*. En variante, ou de manière supplémentaire, les canaux supplémentaires n'apportant aucune augmentation significative de la performance des modèles peuvent être écartés. Le regroupement de canaux peut être également, en variante ou de manière supplémentaire, réalisée en divisant la gamme 390nm-900nm en intervalles dont la largeur correspond à celle des filtres tels que décrit ci-dessus en relation avec les figures 2 et 3. Un seul canal spectral est alors retenu par intervalle. Un nombre final de canaux Où $\lambda_1$, $\lambda_2$, ..., $\lambda_{NF}$ sont ainsi sélectionnés et définissent les longueurs d'onde centrales des filtres spectraux de l'ensemble **36** du système multispectral de la figure 2. Comme cela sera décrit ci-dessous, il est possible d'obtenir une prédiction des classes de grandes précision à l'aide uniquement de 24 canaux, et donc de 24 filtres spectraux.

De manière optionnelle, ayant sélectionné les canaux finaux pour l'application multispectrale et construit le système multispectrale de manière correspondante, un nouvel apprentissage, basé sur l'acquisition de spectres avec le système de la figure 2, est mis en œuvre pour affiner les modèles de prédiction, cet apprentissage étant analogue à celui décrit en relation avec les figures 6 et 7.

De même, il a été décrit la sélection d'un nombre R prédéterminé de canaux discriminant. En variante, ce nombre n'est pas fixé a priori et un critère d'arrêt pour la recherche des créneaux est une stagnation du gain en performance en fonction du nombre de canaux. Si par exemple, l'ajout d'au moins un canal n'augmente pas la performance, par exemple le BCR détaillé ci-après, de plus de X%, alors la recherche de canaux est stoppée, avec par exemple X inférieur ou égale à 2%.

## C. EXEMPLES

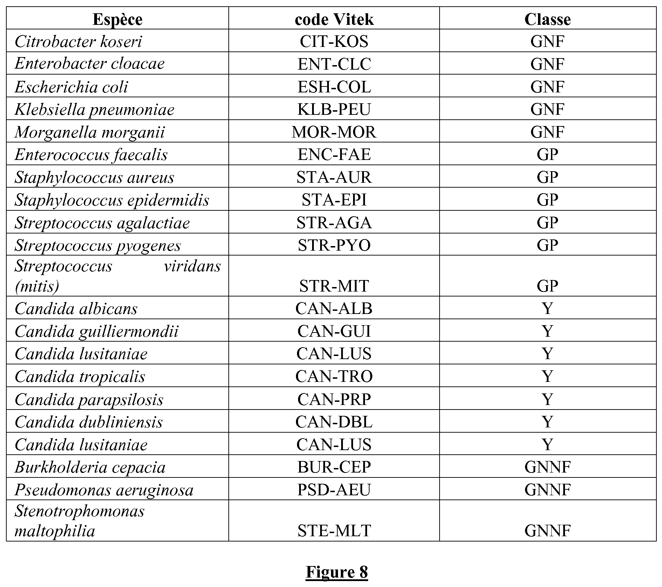

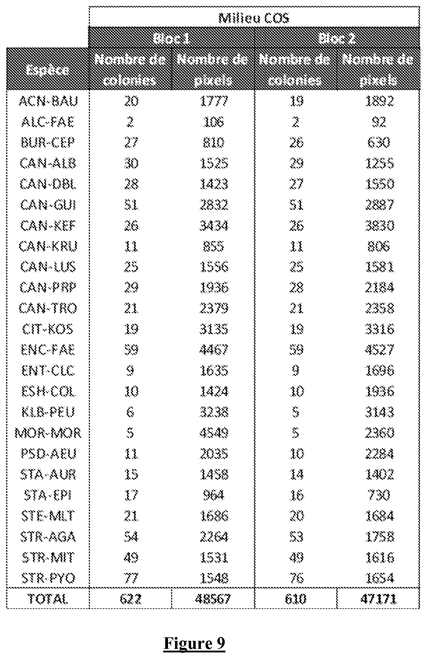

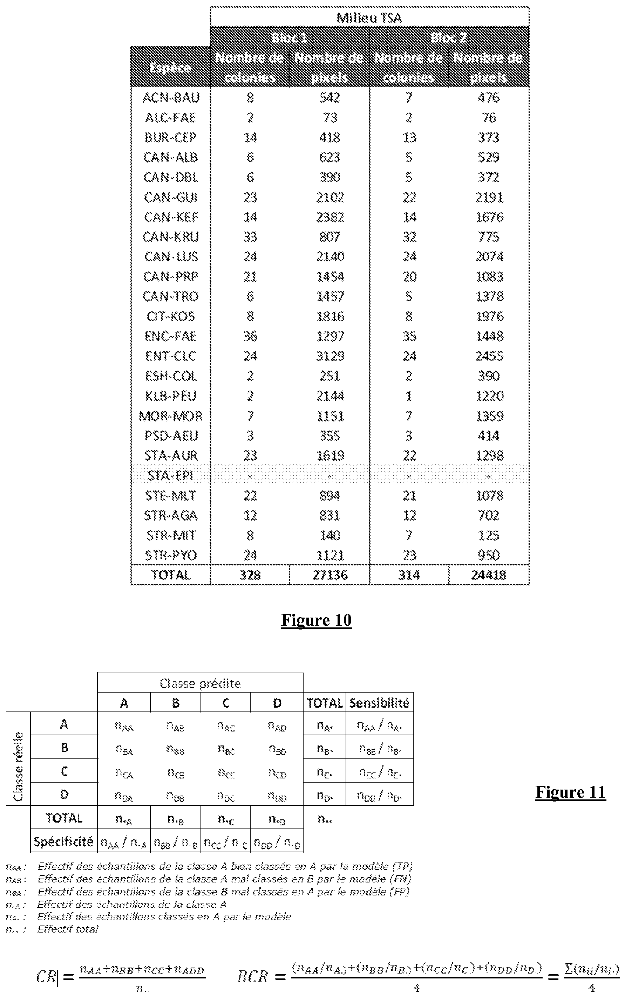

Il va à présent être décrit une application des prédictions des classes Y, GP, GNF et GNN venant d'être décrite. A cette fin, 21 souches bactériennes et de levures sont utilisées, ces espèces microbiennes étant décrite à la figure 8. Ces espèces ont été mises en culture pendant 24 heures sur une gélose COS et une gélose TSA, donnant lieu à une base de données d'apprentissage pour chacun de ces milieux. Le nombre de colonies et de pixels pour chacune des espèces et des milieux sont décrits respectivement à la figure 9 (COS) et à la figure 10 (TSA), le bloc 1 correspondant aux données d'étalonnage et le bloc 2 correspondant aux données de validation croisée.

La performance de la prédiction des classes est avantageusement calculée comme égale à la moyenne des sensibilités des prédictions de classe (taux de spectres bien classés). Ce critère pondéré, également appelé « balance classification rate » ou « BCR », permet de tenir compte des effectifs en pixels qui sont déséquilibrés, ce qui est le cas en raison de la taille des colonies qui est variable en fonction des espèces. Le calcul du BCR est rappelé à la figure 11.

### C.1. RESULTATS COS

#### C.1.1. modèle à plat

La table 1 ci-dessous donne les BCR pour un modèle de prédiction à plat illustré à la figure 7A et pour des modèles de prédiction obtenus à l'aide des relations (7).

Table 1

|  | Etalonnage | Validation croisée |
|---|---|---|
| Y contre GP+GNN+G NF | 85% | 84% |
| GP contre Y+GNN+GNF | 93% | 91% |
| GNN contre Y+GP+GNF | 80% | 80% |
| GNF contre Y+GP+GNN | 99% | 99% |

On note d'emblée à la lecture de la table 1 qu'il est possible de prédire avec précision les différentes souches de bactéries. Notamment, connaissant que le microorganisme à caractériser est une bactérie, il est possible de prédire son type de Gram et son caractère fermentant ou non, en mettant en œuvre une première prédiction GP contre GNN et GNF et une seconde prédiction GNN contre GP et GNF. Ce type de prédiction est notamment utile pour la sélection de consommable pour la réalisation d'un antibiogramme avec la plateformeVitek®2 commercialisée par la demanderesse.

#### C.1.2. Arbre optimal

Les BCR des modèles **110, 112, 114** illustrés à la figure 7C et obtenus à l'aide des relations (7) sont résumés à la table 2.

Table 2

|  | Etalonnage | Validation croisée |
|---|---|---|
| 110 : GNF contre Y + GP+ GNN | 99% | 99% |
| 112 : GP contre Y+GNF | 91% | 90% |
| 114 : Y contre GNF | 86% | 84% |

On note que l'arbre optimal diffère sensiblement de l'arbre phylogénique, l'influence du milieu COS étant vraisemblablement supérieure à l'influence de différences portées par la phylogénie.

Les BCR des modèles **110, 112, 114** illustrés à la figure 7C et obtenus à l'aide de l'approche « step forward » de la figure 4 et des relations (6), avec $R = 24$ pour chacun des modèles, sont résumés à la table 3.

| branch 1 : "GNF" vs "GP+GNN+Y" | | BCR (en %) | | branch 2 : "GP" vs "GNN+Y" | | BCR (en %) | | branch 3 : "GNN" vs "Y" | | BCR (en %) | |
|---|---|---|---|---|---|---|---|---|---|---|---|
| Ordre de sélection | Longueur d'onde | Étalonnage | Validation croisée | Ordre de sélection | Longueur d'onde | Étalonnage | Validation croisée | Ordre de sélection | Longueur d'onde | Étalonnage | Validation croisée |
| 1 | 613.58 | 80.5% | 80.4% | 1 | 634.45 | 78.3% | 78.3% | 1 | 613.58 | 95.1% | 95.1% |
| 2 | 484.16 | 89.0% | 89.0% | 2 | 598.97 | 85.7% | 85.6% | 2 | 661.15 | 95.5% | 95.5% |
| 3 | 634.45 | 93.6% | 93.6% | 3 | 665.76 | 87.9% | 87.7% | 3 | 425.71 | 95.8% | 95.7% |
| 4 | 605.23 | 95.1% | 95.1% | 4 | 630.28 | 89.0% | 88.9% | 4 | 637.75 | 95.9% | 95.9% |
| 5 | 588.53 | 95.9% | 95.9% | 5 | 864.07 | 89.8% | 89.7% | 5 | 334.40 | 96.0% | 95.9% |
| 6 | 640.71 | 96.4% | 96.3% | 6 | 548.87 | 90.3% | 90.1% | 6 | 663.24 | 96.0% | 96.0% |
| 7 | 607.31 | 96.8% | 96.7% | 7 | 482.33 | 91.4% | 91.2% | 7 | 693.90 | 96.1% | 96.0% |
| 8 | 434.06 | 97.1% | 97.1% | 8 | 626.19 | 91.7% | 91.6% | 8 | 421.10 | 96.1% | 96.1% |
| 9 | 603.14 | 97.3% | 97.2% | 9 | 661.58 | 92.1% | 92.0% | 9 | 693.06 | 96.2% | 96.2% |
| 10 | 615.66 | 97.4% | 97.4% | 10 | 584.35 | 92.4% | 92.3% | 10 | 404.84 | 96.3% | 96.2% |
| 11 | 630.28 | 97.7% | 97.7% | 11 | 530.08 | 92.7% | 92.6% | 11 | 655.33 | 96.2% | 96.2% |
| 12 | 657.41 | 97.8% | 97.7% | 12 | 636.54 | 92.9% | 92.8% | 12 | 609.40 | 96.3% | 96.2% |
| 13 | 661.15 | 98.0% | 98.0% | 13 | 603.14 | 93.2% | 93.0% | 13 | 586.44 | 96.3% | 96.3% |
| 14 | 603.05 | 98.1% | 98.1% | 14 | 486.25 | 93.4% | 93.3% | 14 | 584.79 | 96.3% | 96.3% |
| 15 | 642.89 | 98.2% | 98.3% | 15 | 546.78 | 93.6% | 93.5% | 15 | 604.55 | 96.4% | 96.3% |
| 16 | 431.97 | 98.3% | 98.3% | 16 | 361.58 | 93.9% | 93.7% | 16 | 553.04 | 96.4% | 96.3% |
| 17 | 488.33 | 98.3% | 98.3% | 17 | 694.89 | 94.0% | 93.8% | 17 | 578.09 | 96.5% | 96.5% |
| 18 | 638.63 | 98.4% | 98.4% | 18 | 429.39 | 94.1% | 94.0% | 18 | 657.41 | 96.6% | 96.5% |
| 19 | 549.06 | 98.4% | 98.4% | 19 | 632.36 | 94.3% | 94.1% | 19 | 636.54 | 96.6% | 96.6% |
| 20 | 593.97 | 98.5% | 98.5% | 20 | 661.20 | 94.4% | 94.3% | 20 | 550.96 | 96.6% | 96.6% |
| 21 | 866.15 | 98.6% | 98.5% | 21 | 582.27 | 94.5% | 94.4% | 21 | 641.10 | 96.7% | 96.6% |
| 22 | 632.36 | 98.6% | 98.6% | 22 | 586.88 | 94.7% | 94.5% | 22 | 635.66 | 96.7% | 96.6% |
| 23 | 692.70 | 98.7% | 98.7% | 23 | 484.60 | 94.8% | 94.7% | 23 | 576.00 | 96.7% | 96.6% |
| 24 | 629.09 | 98.7% | 98.7% | 24 | 532.17 | 94.8% | 94.7% | 24 | 584.35 | 96.8% | 96.7% |

Table 3

On note à la lecture de la table 3 que le gain en performance est limité à partir du 8e canal pour le premier modèle, et du 4e canal pour le troisième modèle. Pour obtenir une application multispectral à l'aide de 24 filtres spectraux, correspondant aux systèmes de filtre du marché, il est ainsi avantageusement sélectionné respectivement 8 canaux, 14 canaux et 4 canaux pour les premier, deuxième et troisième modèles **110, 112, 114.** Les performances de ce mode de réalisation sont résumés à la table 4.

Table 4

| | Ordre de sélection des canaux les plus discriminant | Longueur d'onde (nm) | BCR |
|---|---|---|---|
| GNF contre Y + GP+ GNN | 1 | 613.58 | 97.10% |
| | 2 | 484.16 | |
| | 3 | 634.45 | |
| | 4 | 605.23 | |
| | 5 | 588.53 | |
| | 6 | 640.71 | |
| | 7 | 607.31 | |
| | 8 | 434.06 | |
| GP contre Y+GNF | 1 | 634.45 | 93.30% |
| | 2 | 598.97 | |
| | 3 | 665.76 | |
| | 4 | 630.28 | |
| | 5 | 864.07 | |

(suite)

| | Ordre de sélection des canaux les plus discriminant | Longueur d'onde (nm) | BCR |
|---|---|---|---|
| | 6 | 548.87 | |
| | 7 | 488.33 | |
| | 8 | 628.19 | |
| | 9 | 661.59 | |
| | 10 | 584.35 | |
| | 11 | 530.08 | |
| | 12 | 636.54 | |
| | 13 | 603.14 | |
| | 14 | 486.25 | |
| Y contre GNF | 1 | 613.58 | |
| | 2 | 651.15 | 95.90% |
| | 3 | 425.71 | |
| | 4 | 617.75 | |

Evidemment d'autres nombres de canaux peuvent être sélectionnés en fonction du nombre de filtres spectraux disponibles pour le système de la figure 2.

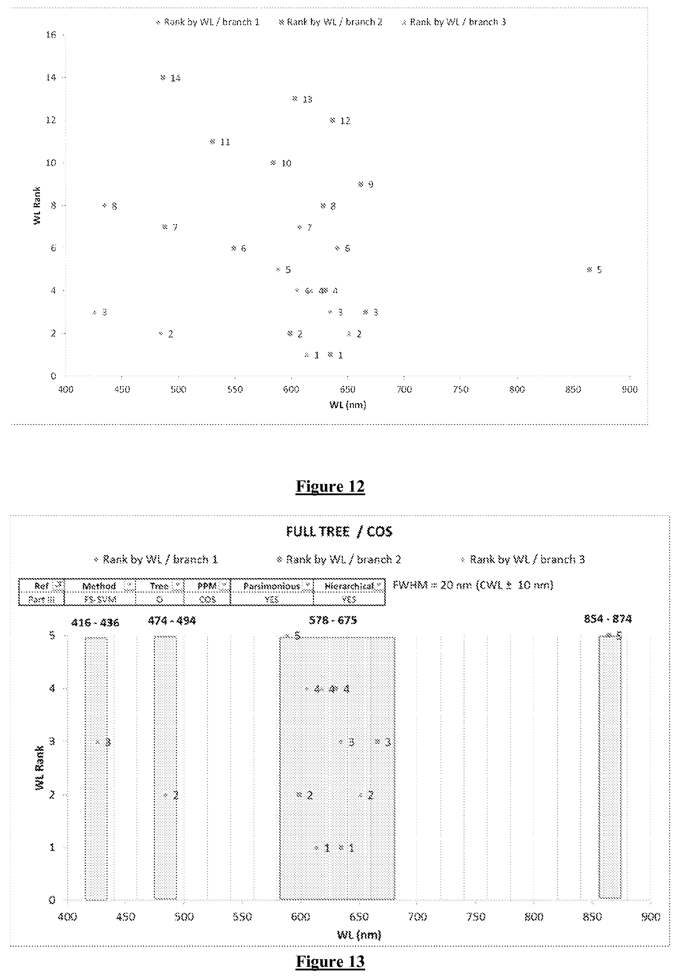

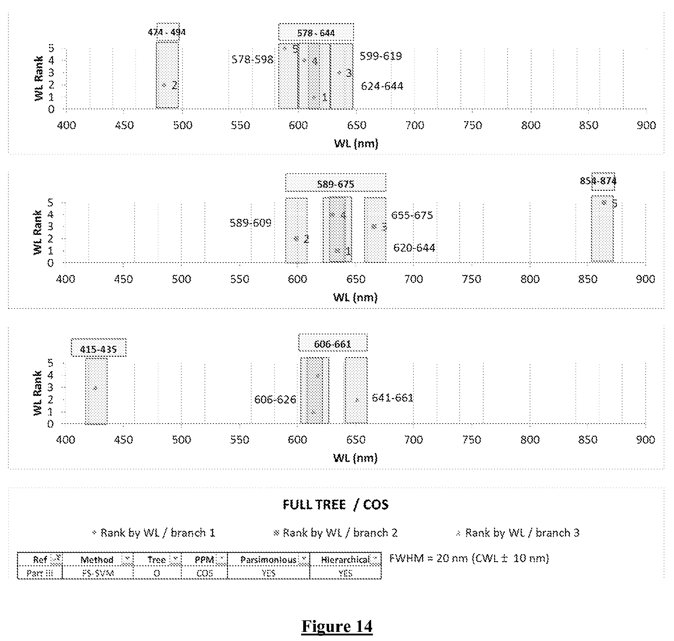

On note par ailleurs que l'approche « step forward » permet de déterminer les gammes spectrales comprenant contenant les informations nécessaires à la prédiction des classes. En se limitant aux cinq premiers canaux de chaque modèle, il est obtenu respectivement des BCR proches ou supérieurs à 90%. La répartition spectrale de ces canaux est illustrée aux figures 12 à 14. On distingue ainsi nettement des bandes spectrales distinctes et distantes les unes des autres de plus de 50 nm. En particulier :

A. les quatre classes Y, GP, GNF et GNN peuvent être prédites efficacement en utilisant uniquement en utilisant les informations spectrale dans une première gamme 415-500nm et une deuxième gamme 535-675 nm. A l'aide de ces gammes uniquement, les BCR sont supérieurs ou égaux à 90%. En limitant la première gamme à 575-675nm, 4 canaux sont utilisés uniquement par modèle pour des BCR proches ou supérieur à 90%. Optionnellement une troisième gamme 850-875 nm, correspondant au canal de rang 5 du deuxième modèle est utilisé. Plus particulièrement, la prédiction dans la première gamme 415-500nm peut être réalisée uniquement sur les gamme 415-440 nm et 470-495 nm. L'invention couvre ainsi tout procédé de prédiction des classes Y, GP, GNF et GNN consistant à acquérir des spectres dans lesdites gammes et à prédire les classes en fonction desdits spectres uniquement dans lesdites gammes. On note par ailleurs que si l'invention permet de distinguer entre les classes Y, GP, GNF et GNN, elle permet donc de distinguer entre des levures et des bactéries, et ce à l'aide des informations spectrales contenues dans les gamme susvisées. L'invention couvre donc également un procédé de prédiction du caractère de levure ou de bactérie d'un microorganisme à caractériser ;

B. la prédiction de la classe GNF contre Y+GP+GNN peut être réalisée efficacement uniquement sur une première gamme 470-500nm et une seconde gamme 575-645nm. L'invention couvre ainsi tout procédé de prédiction des classes la classe GNF consistant à acquérir des spectres dans lesdites gammes et à prédire la classe GNF en fonction desdits spectres uniquement dans lesdites gammes. On notera que lorsque le caractère bactérien du microorganisme à caractériser est déjà connu, la prédiction consiste alors à prédire la classe GNF contre GP et GNN. L'invention couvre donc également ce type de prédiction en se basant uniquement sur les gammes 470-500nm et 575-645nm ;

C. la prédiction de la classe GP contre Y+GNN peut être réalisée efficacement uniquement sur la première gamme 535-675 nm, et plus particulièrement sur la gamme 585-675nm, et la deuxième gamme 850-875nm. L'invention couvre ainsi tout procédé de prédiction des classes la classe GP consistant à acquérir des spectres dans lesdites gammes et à prédire la classe GP en fonction desdits spectres uniquement dans lesdites gammes. On notera que lorsque le caractère bactérien du microorganisme à caractériser est déjà connu, la prédiction consiste alors à prédire la classe GP contre les classes GNF et GNN, et par conséquent par la classe GP contre la classe des bactéries à Gram négatif (GN). L'invention couvre donc également ce type de prédiction en se basant uniquement sur les gammes 535-675 nm, et plus particulièrement sur la gamme 585-675nm, et la gamme 850-875nm;

D. En combinant les prédictions décrites aux points B et C ci-dessous, on note ainsi qu'avec trois gammes, et connaissant le caractère bactérien du microorganisme à caractérisé, il est possible de déterminer si une colonie bactérienne est GP, GNF ou GNN. Ce type de prédiction est notamment utile pour la sélection de consommable pour la réalisation d'un antibiogramme avec la plateformeVitek®2 commercialisée par la demanderesse.

C.1.3. Arbre phylogénique

Les BCR des modèles **100, 102, 104** illustrés à la figure 7B et obtenus à l'aide des relations (7) sont résumés à la table 5.

Table 5

|  | Etalonnage | Validation croisée |
|---|---|---|
| 100 : Y contre GP+GNN+G NF | 85% | 84% |
| 102 : GP contre GNN+GNF | 95% | 96% |
| 104 : GNF contre GNN | 97% | 97% |

C.2. RESULTATS TSA

C.2.1. modèle à plat

La table 1 ci-dessous donne les BCR pour un modèle de prédiction à plat illustré à la figure 7A et pour des modèles de prédiction obtenus à l'aide des relations (7).

Table 6

|  | Etalonnage | Validation croisée |
|---|---|---|
| Y contre GP+GNN+GNF | 89% | 88% |
| GP contre Y+GNN+GNF | 91% | 90% |
| GNN contre Y+GP+GNF | 75% | 73% |
| GNF contre Y+GP+GNN | 90% | 88% |

C.2.2. Arbre optimal

Les BCR des modèles **110, 112, 114** illustrés à la figure 7C et obtenus à l'aide des relations (7) sont résumés à la table 7.

Table 7

|  | Etalonnage | Validation croisée |
|---|---|---|
| 110 : GP contre Y +GNF+GNN | 91% | 90% |
| 112 : Y contre GNF+GNN | 94% | 93% |
| 114 : GNF contre GNN | 82% | 81% |

On note que l'arbre optimal diffère sensiblement de l'arbre phylogénique, l'influence du milieu COS étant vraisemblablement supérieure à l'influence de différences portées par la phylogénie.

Les BCR des modèles **110, 112, 114** illustrés à la figure 7C et obtenus à l'aide de l'approche « step forward » de la figure 5 et des relations (6), avec R = 24 pour chacun des modèles, sont résumés à la table 8.

| Ordre de sélection | Longueur d'onde | Etalonnage | Validation croisée | Ordre de sélection | Longueur d'onde | Etalonnage | Validation croisée | Ordre de sélection | Longueur d'onde | Etalonnage | Validation croisée |
|---|---|---|---|---|---|---|---|---|---|---|---|
| 1 | 482.07 | 65.8% | 65.9% | 1 | 431.97 | 86.4% | 86.4% | 1 | 398.57 | 58.5% | 58.5% |
| 2 | 805.69 | 72.1% | 71.7% | 2 | 428.85 | 87.0% | 87.5% | 2 | 396.49 | 60.4% | 60.5% |
| 3 | 831.97 | 74.6% | 74.7% | 3 | 266.15 | 88.9% | 88.1% | 3 | 400.65 | 61.9% | 61.2% |
| 4 | 557.02 | 76.5% | 76.2% | 4 | 558.30 | 90.3% | 89.3% | 4 | 384.43 | 62.7% | 62.5% |
| 5 | 859.89 | 82.4% | 81.3% | 5 | 868.74 | 90.7% | 90.3% | 5 | 434.06 | 63.0% | 62.0% |
| 6 | 703.50 | 83.3% | 83.3% | 6 | 542.61 | 91.1% | 90.7% | 6 | 510.85 | 65.5% | 65.1% |
| 7 | 479.98 | 85.1% | 85.0% | 7 | 557.12 | 91.6% | 91.0% | 7 | 494.65 | 73.9% | 72.3% |
| 8 | 481.06 | 85.7% | 85.7% | 8 | 434.86 | 91.6% | 91.5% | 8 | 522.91 | 75.3% | 73.5% |
| 9 | 944.46 | 76.3% | 85.3% | 9 | 509.31 | 91.5% | 91.6% | 9 | 500.85 | 76.6% | 74.2% |
| 10 | 578.53 | 86.7% | 86.7% | 10 | 433.18 | 92.0% | 91.8% | 10 | 525.72 | 77.0% | 74.5% |
| 11 | 427.80 | 87.1% | 87.1% | 11 | 427.80 | 92.1% | 92.0% | 11 | 366.78 | 77.2% | 75.0% |
| 12 | 583.30 | 87.6% | 87.6% | 12 | 561.39 | 92.2% | 92.1% | 12 | 726.32 | 77.9% | 75.9% |
| 13 | 488.31 | 88.1% | 88.1% | 13 | 409.41 | 92.4% | 92.2% | 13 | 651.33 | 76.6% | 76.6% |
| 14 | 486.13 | 88.4% | 88.4% | 14 | 400.65 | 93.6% | 93.4% | 14 | 525.69 | 78.8% | 77.2% |
| 15 | 576.56 | 88.8% | 88.8% | 15 | 546.69 | 92.7% | 92.6% | 15 | 526.69 | 79.1% | 77.5% |
| 16 | 484.36 | 88.9% | 88.9% | 16 | 891.29 | 92.5% | 92.6% | 16 | 505.03 | 79.5% | 77.5% |
| 17 | 482.75 | 89.2% | 89.1% | 17 | 734.65 | 93.3% | 92.7% | 17 | 572.90 | 79.5% | 78.5% |
| 18 | 425.71 | 89.3% | 89.3% | 18 | 557.41 | 93.4% | 93.1% | 18 | 632.45 | 79.7% | 78.9% |
| 19 | 560.76 | 89.4% | 89.4% | 19 | 436.35 | 93.5% | 93.3% | 19 | 657.41 | 79.3% | 78.3% |
| 20 | 423.62 | 89.6% | 89.5% | 20 | 368.11 | 93.7% | 93.4% | 20 | 549.06 | 79.9% | 78.3% |
| 21 | 348.43 | 89.8% | 89.7% | 21 | 742.60 | 93.5% | 93.5% | 21 | 618.14 | 80.4% | 78.7% |
| 22 | 488.25 | 89.9% | 89.8% | 22 | 876.23 | 93.5% | 93.6% | 22 | 847.37 | 80.3% | 78.9% |
| 23 | 582.24 | 90.0% | 89.9% | 23 | 734.56 | 94.0% | 92.7% | 23 | 657.85 | 80.1% | 79.0% |
| 24 | 876.52 | 91.2% | 90.0% | 24 | 368.50 | 94.2% | 93.2% | 24 | 796.22 | 80.2% | 79.2% |

Branch 1 : "GP" versus "GNF+GNN"      Branch 2 : "Y" versus "GNN+GNF"      Branch 3 : "GNF" versus "GNN"

Table 8

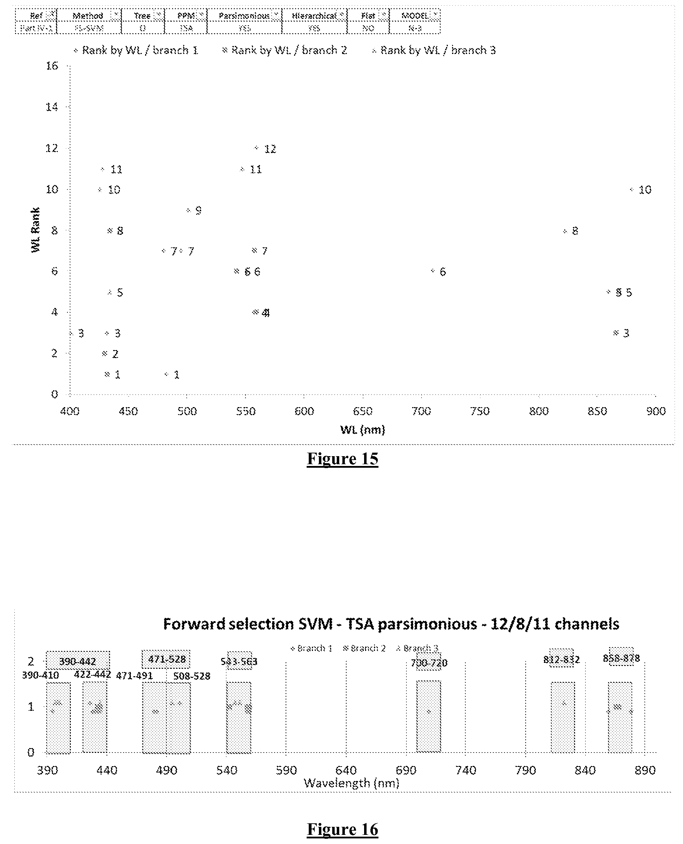

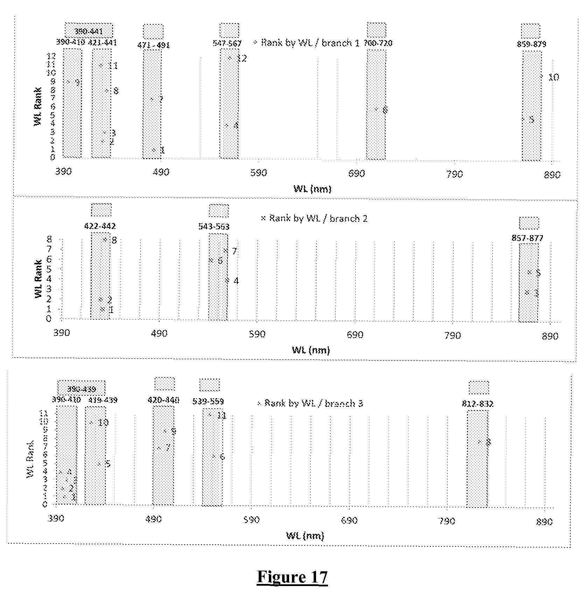

Les figures 15 à 17 illustrent la répartition spectrale des principaux canaux des branches, dans un même graphique (figure 15), dans une approche parcimonieuse avec 12, 8 et 11 canaux pour les premier, deuxième et troisième modèles **110, 112** et **114,** (figure 16) et par modèle (figure 17).

C.2.3. Arbre phylogénique

Les BCR des modèles **100, 102, 104** illustrés à la figure 7B et obtenus à l'aide des relations (7) sont résumés à la table 9.

Table 9

|  | Etalonnage | Validation croisée |
|---|---|---|
| 100 : Y contre GP+GNN+GNF | 89% | 88% |
| 102 : GP contre GNN+GNF | 92% | 92% |
| 104 : GNF contre GNN | 82% | 81% |

Les BCR des modèles **100, 102, 104** illustrés à la figure 7B et obtenus à l'aide de l'approche « step forward » de la figure 4 et des relations (6), avec R = 24 pour chacun des modèles, sont résumés à la table 10.

**1ère branche : "Y" versus "GNF+GNN+GP"**

| Ordre de sélection | Longueur d'onde | BCR (en %) | |
| --- | --- | --- | --- |
| | | Etalonnage | Validation croisée |
| 1 | 475,81 | 84,3% | 84,3% |
| 2 | 540,52 | 84,8% | 84,7% |
| 3 | 717,95 | 85,7% | 85,9% |
| 4 | 486,25 | 85,8% | 86,1% |
| 5 | 657,41 | 86,2% | 86,3% |
| 6 | 701,25 | 86,3% | 86,6% |
| 7 | 845,28 | 86,7% | 86,8% |
| 8 | 680,37 | 86,8% | 86,9% |
| 9 | 490,42 | 86,9% | 87,0% |
| 10 | 655,33 | 87,1% | 87,2% |
| 11 | 684,55 | 87,2% | 87,4% |
| 12 | 686,64 | 87,3% | 87,4% |
| 13 | 659,50 | 87,5% | 87,5% |
| 14 | 803,53 | 87,6% | 87,7% |
| 15 | 728,38 | 87,6% | 87,7% |
| 16 | 682,46 | 87,7% | 87,8% |
| 17 | 582,27 | 87,7% | 87,9% |
| 18 | 546,78 | 87,8% | 87,9% |
| 19 | 651,15 | 87,8% | 88,0% |
| 20 | 661,59 | 88,0% | 88,1% |
| 21 | 571,83 | 88,0% | 88,1% |
| 22 | 454,93 | 88,0% | 88,1% |
| 23 | 617,75 | 88,0% | 88,1% |
| 24 | 400,66 | 88,0% | 88,2% |

**2ème branche : "GP" versus "GNN+GNF"**

| Ordre de sélection | Longueur d'onde | BCR (en %) | |
| --- | --- | --- | --- |
| | | Etalonnage | Validation croisée |
| 1 | 431,97 | 75,5% | 75,6% |
| 2 | 427,80 | 77,2% | 77,2% |
| 3 | 477,90 | 80,3% | 80,3% |
| 4 | 434,06 | 81,8% | 81,8% |
| 5 | 891,20 | 83,3% | 83,2% |
| 6 | 559,30 | 87,5% | 87,4% |
| 7 | 872,41 | 88,2% | 88,2% |
| 8 | 486,25 | 88,8% | 88,7% |
| 9 | 394,40 | 89,2% | 89,2% |
| 10 | 429,89 | 89,8% | 89,7% |
| 11 | 398,57 | 90,1% | 90,1% |
| 12 | 438,24 | 90,3% | 90,3% |
| 13 | 880,76 | 90,5% | 90,5% |
| 14 | 465,37 | 90,7% | 90,7% |
| 15 | 396,49 | 90,9% | 90,9% |
| 16 | 517,56 | 91,1% | 91,0% |
| 17 | 557,22 | 91,3% | 91,3% |
| 18 | 406,92 | 91,5% | 91,5% |
| 19 | 421,54 | 91,6% | 91,6% |
| 20 | 436,15 | 91,8% | 91,7% |
| 21 | 511,29 | 91,9% | 91,9% |
| 22 | 882,85 | 92,1% | 92,0% |
| 23 | 425,71 | 92,1% | 92,1% |
| 24 | 423,62 | 92,3% | 92,3% |

**3ème branche : "GNF" versus "GNN"**

| Ordre de sélection | Longueur d'onde | BCR (en %) | |
| --- | --- | --- | --- |
| | | Etalonnage | Validation croisée |
| 1 | 398,57 | 58,5% | 58,3% |
| 2 | 396,49 | 60,4% | 60,6% |
| 3 | 400,66 | 61,9% | 61,8% |
| 4 | 394,40 | 62,7% | 62,6% |
| 5 | 434,06 | 63,0% | 63,0% |
| 6 | 550,96 | 68,5% | 67,1% |
| 7 | 494,60 | 73,9% | 72,3% |
| 8 | 822,32 | 75,3% | 73,5% |
| 9 | 500,86 | 76,0% | 74,2% |
| 10 | 425,71 | 77,0% | 74,9% |
| 11 | 546,78 | 77,8% | 75,6% |
| 12 | 726,30 | 77,9% | 75,9% |
| 13 | 655,33 | 78,6% | 76,6% |
| 14 | 626,10 | 78,8% | 77,2% |
| 15 | 826,49 | 79,1% | 77,4% |
| 16 | 505,03 | 79,5% | 77,8% |
| 17 | 874,50 | 79,5% | 78,0% |
| 18 | 634,45 | 79,7% | 78,0% |
| 19 | 657,41 | 79,8% | 78,4% |
| 20 | 649,06 | 79,9% | 78,5% |
| 21 | 818,14 | 80,2% | 78,7% |
| 22 | 847,37 | 80,2% | 78,9% |
| 23 | 667,85 | 80,3% | 79,0% |
| 24 | 786,83 | 80,2% | 79,1% |

Table 10

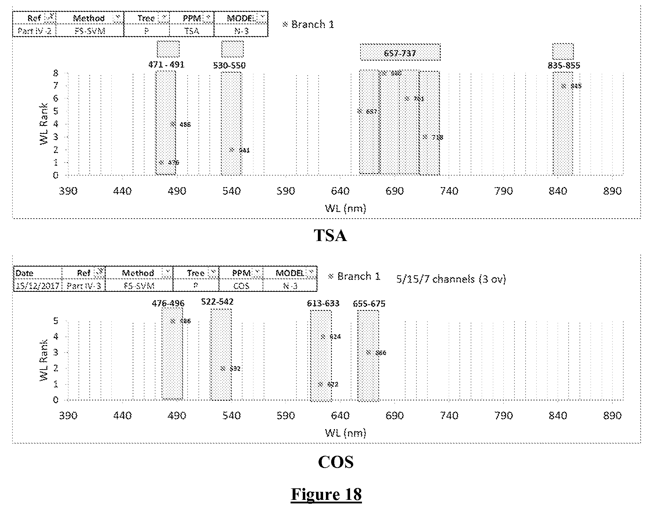

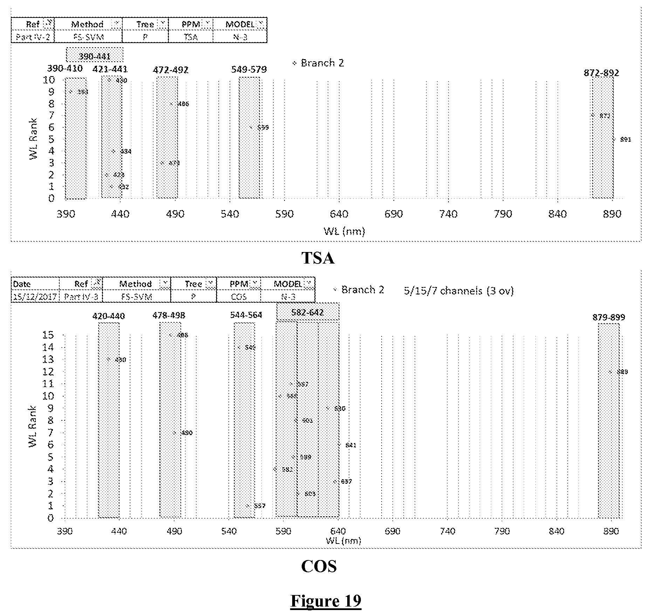

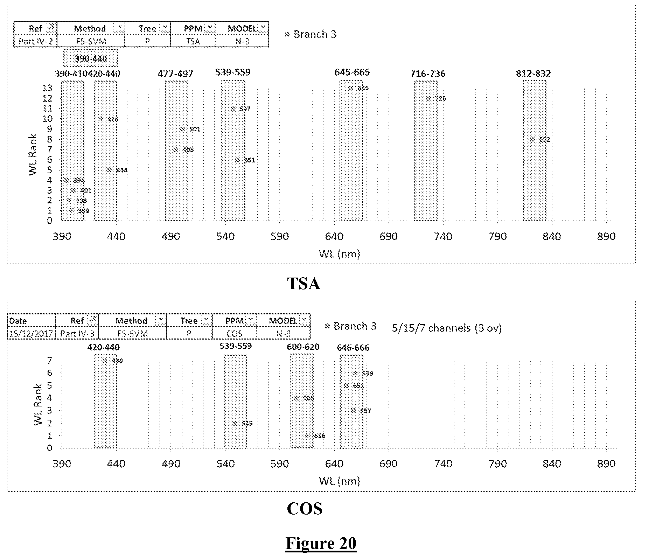

Les figures 18 à 20 illustrent la répartition spectrale des principaux canaux spectraux, modèle par modèle, avec le milieu TSA en haut de chacune de ces figures et en comparaison avec le milieu COS en bas de chacune des figures.

**Revendications**

1. Procédé de caractérisation d'un microorganisme sous forme de colonie cultivée dans un milieu nutritif non chromogène et non fluorescent, comportant :

   - l'éclairage dans la gamme de longueurs d'onde 390nm-900nm de la colonie du microorganisme ayant une réponse électromagnétique naturelle dans ladite gamme;
   - l'acquisition, dans ladite gamme, d'une intensité lumineuse réfléchie par, ou transmise au travers de, la colonie du microorganisme éclairée, l'acquisition de l'intensité lumineuse comportant l'acquisition d'une image hyperspectrale ou multispectrale de la colonie du microorganisme, et l'intensité lumineuse étant déterminée en fonction d'au moins un pixel de ladite image correspondant à la colonie ; et
   - la détermination du microorganisme comme étant une levure ou une souche bactérienne en fonction de l'intensité lumineuse acquise dans ladite gamme.

2. Procédé selon la revendication 1, comportant en outre la détection du type de Gram et du caractère fermentant d'une souche bactérienne, comportant :

   - l'éclairage dans la gamme de longueurs d'onde 390nm-900nm d'au moins une bactérie de ladite souche ayant une réponse électromagnétique naturelle dans ladite gamme;
   - l'acquisition, dans ladite gamme, d'une intensité lumineuse réfléchie par, ou transmise au travers de, ladite bactérie éclairée; et

   la détermination du type de Gram et du caractère fermentant de la souche bactérienne en fonction de l'intensité lumineuse acquise dans ladite gamme

3. Procédé selon la revendication 2, dans lequel la détermination du type de Gram et du caractère fermentant comporte l'application d'une classification prédisant si l'intensité lumineuse acquise est celle d'une souche bactérienne de type Gram négatif et fermentante.

4. Procédé selon la revendication 2 ou 3, dans lequel la détermination du type de Gram et du caractère fermentant comporte l'application d'une classification prédisant si l'intensité lumineuse acquise est celle d'une souche bactérienne du type Gram positif ou l'application d'une classification prédisant si l'intensité lumineuse acquise est celle d'une souche bactérienne du type Gram négatif.

5. Procédé selon la revendication 3, dans lequel :

   - l'éclairage et l'acquisition sont réalisés directement sur un échantillon comprenant une colonie de la souche bactérienne et un milieu nutritif sur lequel ladite colonie a poussé, le milieu nutritif étant une gélose au sang, en particulier un columbia de sang de mouton ;
   - si l'intensité lumineuse acquise n'est pas celle d'une souche bactérienne de type Gram négatif et fermentante, la détermination du type de Gram et du caractère fermentant comporte en outre l'application d'une classification prédisant si l'intensité lumineuse acquise est celle d'une souche bactérienne du type Gram positif.

6. Procédé selon la revendication 5, dans lequel, si l'intensité lumineuse acquise n'est pas celle d'une souche bactérienne de type Gram positif, la détermination du type de Gram et du caractère fermentant comporte en outre l'application d'une classification prédisant si l'intensité lumineuse acquise est celle d'une levure.

7. Procédé selon la revendication 6, dans lequel

   - la classification prédisant si l'intensité lumineuse acquise est celle d'une souche bactérienne de type Gram négatif et fermentante est une classification distinguant l'intensité lumineuse des souches bactériennes de type Gram négatif et fermentantes de l'intensité lumineuse de l'ensemble constitué des souches bactérienne de type négatif et non fermentantes, des souches bactérienne de type Gram positif et des levures ;
   - la classification prédisant si l'intensité lumineuse acquise est celle d'une souche bactérienne de type Gram positif est une classification distinguant l'intensité lumineuse des souches bactérienne de type Gram positif de l'intensité lumineuse de l'ensemble constitué des souches bactérienne de type négatif et non fermentantes et des levures.
   - la classification prédisant si l'intensité lumineuse acquise est celle d'une levure est une classification distinguant l'intensité lumineuse des levures de l'intensité lumineuse de l'ensemble constitué des souches bactérienne de type Gram négatif et non fermentantes.

8. Procédé selon la revendication 2, dans lequel, si l'intensité lumineuse acquise n'est pas celle d'une levure, la détermination du type de Gram et du caractère fermentant comporte en outre l'application d'une classification prédisant si l'intensité lumineuse acquise est celle d'une souche bactérienne de type Gram positif.

9. Procédé selon la revendication 8, dans lequel, si l'intensité lumineuse acquise n'est pas celle d'une souche bactérienne de type Gram positif, la détermination du type de Gram et du caractère fermentant comporte en outre l'application d'une classification prédisant si l'intensité lumineuse est celle d'une souche bactérienne de type Gram négatif et fermentante ou d'une souche bactérienne de type Gram négatif et non fermentante.

10. Procédé selon la revendication 9, dans lequel :

    - la classification prédisant si l'intensité lumineuse acquise est celle d'une levure est une classification distinguant l'intensité lumineuse des levures de l'intensité lumineuse de l'ensemble constitué des souches bactériennes de type Gram positif, des souches bactériennes de type Gram négatif et non fermentantes et des souches bactériennes de type Gram négatif et fermentantes ;
    - la classification prédisant si l'intensité lumineuse est celle d'une souche bactérienne de type Gram positif est une classification distinguant l'intensité lumineuse des souches bactériennes de type Gram positif de l'intensité lumineuse de l'ensemble constitué des souches bactériennes de type Gram négatif et non fermentantes et des souches bactériennes de type Gram négatif et fermentantes ;
    - la classification si l'intensité lumineuse est celle d'une souche bactérienne de type Gram négatif et fermentante ou d'une souche bactérienne de type Gram négatif et non fermentante est une classification distinguant l'intensité lumineuse des souches bactériennes de type Gram négatif et non fermentantes de l'intensité lumineuse du groupe constitué des souches bactériennes de type Gram négatif et fermentantes ;

**11.** Procédé selon la revendication 4, dans lequel :

- l'éclairage et l'acquisition sont réalisés directement sur un échantillon comprenant une colonie de la souche bactérienne et un milieu nutritif sur lequel ladite colonie a poussé, le milieu nutritif étant une gélose Tryptone-Soja ;
- si l'intensité lumineuse acquise n'est pas celle d'une souche bactérienne de type Gram positif, la détermination du type de Gram et du caractère fermentant comporte en outre l'application d'une classification prédisant si l'intensité lumineuse acquise est celle d'une levure.

**12.** Procédé selon la revendication 11, dans lequel, si l'intensité lumineuse acquise n'est pas celle d'une levure, la détermination du type de Gram et du caractère fermentant comporte en outre l'application d'une classification prédisant si l'intensité lumineuse est celle d'une souche bactérienne de type Gram négatif et fermentante ou d'une souche bactérienne de type Gram négatif et non fermentante.

**13.** Procédé selon la revendication 12, dans lequel :

- la classification prédisant si l'intensité lumineuse est celle d'une souche bactérienne de type Gram positif est une classification distinguant l'intensité lumineuse des souches bactériennes de type Gram positif de l'intensité lumineuse de l'ensemble constitué des souches bactériennes de type Gram négatif et non fermentantes, des souches bactériennes de type Gram négatif et fermentantes et des levures;
- la classification prédisant si l'intensité lumineuse acquise est celle levure est une classification distinguant l'intensité lumineuse des levures de l'intensité de l'ensemble constitué des souches bactérienne de type négatif et non fermentantes et des souches bactérienne de type négatif et fermentantes;
- la classification si l'intensité lumineuse est celle d'une souche bactérienne de type Gram négatif et fermentante ou d'une souche bactérienne de type Gram négatif et non fermentante est une classification distinguant l'intensité lumineuse des souches bactériennes de type Gram négatif et non fermentantes de l'intensité lumineuse du groupe constitué des souches bactériennes de type Gram négatif et fermentantes .

**14.** Procédé selon l'une des revendications 3 à 13, dans lequel chaque classification est apprise sur des images hyperspectrales de la gamme 390nm-900 nm et selon une approche consistant à augmenter pas-à-pas un ensemble de canaux spectraux utilisés dans la classification jusqu'à obtention d'un seuil de précision prédéterminé ou d'un nombre maximal de canaux prédéterminé.

**15.** Procédé selon la revendications 3, dans lequel la première classification distingue les intensités lumineuses en fonction de la gamme de longueurs d'onde 470nm-500 nm et de la gamme de longueurs d'onde 575-645 nm uniquement.

**16.** Procédé selon la revendication 4, dans lequel la seconde classification distingue les intensités lumineuses en fonction de la gamme de longueurs d'onde 535nm - 675 nm et de la gamme de longueurs d'onde 850 nm-875 nm uniquement.

**17.** Procédé selon l'une des revendications 5 à 7, dans lequel la seconde classification distingue les intensités lumineuses en fonction de la gamme de longueurs d'onde 415nm - 500nm et de la gamme de longueurs d'onde 535 nm - 675 nm uniquement.

**18.** Procédé selon l'une quelconque des revendications précédentes, dans lequel l'intensité lumineuse est acquise sur un nombre de canaux spectraux inférieur ou égal à 24.

**19.** Procédé selon l'une quelconque des revendications 5 à 7, dans lequel l'intensité lumineuse est acquise sur un nombre de canaux spectraux inférieur ou égal à 5 pour chacune des première et seconde classifications, et de préférence inférieur ou égal à 4.

**20.** Procédé selon l'une quelconque des revendications précédentes, dans lequel la détermination du type de Gram et du caractère fermentant est réalisée en fonction de l'intensité lumineuse de chaque pixel d'un ensemble de pixels de la colonie, et dans laquelle le type de Gram et le caractère fermentant sont déterminés par un vote majoritaire des résultats desdites premières détections.

**21.** Procédé selon la revendication 20, dans lequel le vote majoritaire est un vote à 70% des pixels ou plus ou un vote à la majorité simple.

22. Système de caractérisation d'un microorganisme sous forme de colonie cultivée dans un milieu nutritif non chromogène et non fluorescent, comprenant :

- un éclairage configuré pour éclairer, dans la gamme de longueurs d'onde 390nm-900 nm, la colonie du microorganisme;
- un capteur configuré pour acquérir, dans la gamme 390nm-900nm, une image hyperspectrale ou multispectrale réfléchie par, ou transmise au travers de, la colonie du microorganisme éclairé; et
- une unité informatique configurée pour

  ○ déterminer une intensité lumineuse dans la gamme 390nm-900nm en fonction d'au moins un pixel de ladite image hyperspectrale ou multispectrale de la colonie du microorganisme ; et
  ○ déterminer le microorganisme comme étant une levure ou une souche bactérienne en fonction de l'intensité lumineuse acquise dans ladite gamme.

23. Système selon la revendication 22, configuré pour mettre en œuvre un procédé conforme à l'une des revendications 2 à 21.

24. Système selon la revendication 22 ou 23, configuré pour éclairer, et acquérir l'image de, un échantillon comprenant une colonie de microorganismes et un milieu nutritif sur lequel ladite colonie a poussé, en particulier une boite de Pétri.

**Patentansprüche**

1. Verfahren zur Charakterisierung eines Mikroorganismus in Form einer in einem nicht chromogenen und nicht fluoreszierenden Nährmedium kultivierten Kolonie, umfassend:

- das Beleuchten der Kolonie des Mikroorganismus in dem Wellenlängenbereich 390 nm-900 nm mit einer natürlichen elektromagnetischen Antwort in dem Bereich;
- das Erfassen, in dem Bereich, einer von der beleuchteten Kolonie des Mikroorganismus reflektierten oder durch sie hindurch transmittierten Lichtintensität, wobei das Erfassen der Lichtintensität das Erfassen eines hyperspektralen oder multispektralen Bilds der Kolonie des Mikroorganismus umfasst und wobei die Lichtintensität in Abhängigkeit von mindestens einem Pixel des der Kolonie entsprechenden Bilds bestimmt wird; und
- das Bestimmen des Mikroorganismus als eine Hefe oder ein Bakterienstamm in Abhängigkeit von der in dem Bereich erfassten Lichtintensität.

2. Verfahren nach Anspruch 1, umfassend ferner das Detektieren des Gram-Typs und des fermentierenden Charakters eines Bakterienstamms, umfassend:

- das Beleuchten mindestens einer Bakterie des Stamms in dem Wellenlängenbereich 390 nm-900 nm mit einer natürlichen elektromagnetischen Antwort in dem Bereich;
- das Erfassen, in dem Bereich, einer von der beleuchteten Bakterie reflektierten oder durch sie hindurch transmittierten Lichtintensität; und

das Bestimmen des Gram-Typs und des fermentierenden Charakters des Bakterienstamms in Abhängigkeit von der in dem Bereich erfassten Lichtintensität.

3. Verfahren nach Anspruch 2, wobei das Bestimmen des Gram-Typs und des fermentierenden Charakters das Anwenden einer Klassifikation umfasst, die vorhersagt, ob die erfasste Lichtintensität diejenige eines gramnegativen und fermentierenden Bakterienstamms ist.

4. Verfahren nach Anspruch 2 oder 3, wobei das Bestimmen des Gram-Typs und des fermentierenden Charakters das Anwenden einer Klassifikation umfasst, die vorhersagt, ob die erfasste Lichtintensität diejenige eines grampositiven Bakterienstamms ist, oder das Anwenden einer Klassifikation, die vorhersagt, ob die erfasste Lichtintensität diejenige eines gramnegativen Bakterienstamms ist.

5. Verfahren nach Anspruch 3, wobei:

- das Beleuchten und das Erfassen direkt an einer Probe ausgeführt werden, die eine Kolonie des Bakterienstamms und ein Nährmedium, auf dem die Kolonie gewachsen ist, umfasst, wobei das Nährmedium ein Blutagar ist, insbesondere ein Columbia-Agar aus Schafblut;
- wenn die erfasste Lichtintensität nicht diejenige eines gramnegativen und fermentierenden Bakterienstamms ist, das Bestimmen des Gram-Typs und des fermentierenden Charakters ferner das Anwenden einer Klassifikation umfasst, die vorhersagt, ob die erfasste Lichtintensität diejenige eines grampositiven Bakterienstamms ist.

6. Verfahren nach Anspruch 5, wobei, wenn die erfasste Lichtintensität nicht diejenige eines grampositiven Bakterienstamms ist, das Bestimmen des Gram-Typs und des fermentierenden Charakters ferner das Anwenden einer Klassifikation umfasst, die vorhersagt, ob die erfasste Lichtintensität diejenige einer Hefe ist.

7. Verfahren nach Anspruch 6, wobei

- die Klassifikation, die vorhersagt, ob die erfasste Lichtintensität diejenige eines gramnegativen und fermentierenden Bakterienstamms ist, eine Klassifikation ist, die die Lichtintensität der gramnegativen und fermentierenden Bakterienstämme von der Lichtintensität der Menge, die aus den negativen und nicht fermentierenden Bakterienstämmen, den grampositiven Bakterienstämmen und den Hefen besteht, unterscheidet;
- die Klassifikation, die vorhersagt, ob die erfasste Lichtintensität diejenige eines grampositiven Bakterienstamms ist, eine Klassifikation ist, die die Lichtintensität der grampositiven Bakterienstämme von der Lichtintensität der Menge, die aus den negativen und nicht fermentierenden Bakterienstämmen und den Hefen besteht, unterscheidet;
- die Klassifikation, die vorhersagt, ob die erfasste Lichtintensität diejenige einer Hefe ist, eine Klassifikation ist, die die Lichtintensität der Hefen von der Lichtintensität der Menge, die aus den gramnegativen und nicht fermentierenden Bakterienstämmen besteht, unterscheidet.

8. Verfahren nach Anspruch 2, wobei, wenn die erfasste Lichtintensität nicht diejenige einer Hefe ist, das Bestimmen des Gram-Typs und des fermentierenden Charakters ferner das Anwenden einer Klassifikation umfasst, die vorhersagt, ob die erfasste Lichtintensität diejenige eines grampositiven Bakterienstamms ist.

9. Verfahren nach Anspruch 8, wobei, wenn die erfasste Lichtintensität nicht diejenige eines grampositiven Bakterienstamms ist, das Bestimmen des Gram-Typs und des fermentierenden Charakters ferner das Anwenden einer Klassifikation umfasst, die vorhersagt, ob die Lichtintensität diejenige eines gramnegativen und fermentierenden Bakterienstamms oder eines gramnegativen und nicht fermentierenden Bakterienstamms ist.

10. Verfahren nach Anspruch 9, wobei:

- die Klassifikation, die vorhersagt, ob die erfasste Lichtintensität diejenige einer Hefe ist, eine Klassifikation ist, die die Lichtintensität der Hefen von der Lichtintensität der Menge, die aus den grampositiven Bakterienstämmen, den gramnegativen und nicht fermentierenden Bakterienstämmen und den gramnegativen und fermentierenden Bakterienstämmen besteht, unterscheidet;
- die Klassifikation, die vorhersagt, ob die Lichtintensität diejenige eines grampositiven Bakterienstamms ist, eine Klassifikation ist, die die Lichtintensität der grampositiven Bakterienstämme von der Lichtintensität der Menge, die aus den gramnegativen und nicht fermentierenden Bakterienstämmen und den gramnegativen und fermentierenden Bakterienstämmen besteht, unterscheidet;
- die Klassifikation, ob die Lichtintensität diejenige eines gramnegativen und fermentierenden Bakterienstamms oder eines gramnegativen und nicht fermentierenden Bakterienstamms ist, eine Klassifikation ist, die die Lichtintensität der gramnegativen und nicht fermentierenden Bakterienstämme von der Lichtintensität der Gruppe, die aus den gramnegativen und fermentierenden Bakterienstämmen besteht, unterscheidet.

11. Verfahren nach Anspruch 4, wobei:

- das Beleuchten und das Erfassen direkt an einer Probe ausgeführt werden, die eine Kolonie des Bakterienstamms und ein Nährmedium, auf dem die Kolonie gewachsen ist, umfasst, wobei das Nährmedium ein Trypton-Soja-Agar ist;
- wenn die erfasste Lichtintensität nicht diejenige eines grampositiven Bakterienstamms ist, das Bestimmen des Gram-Typs und des fermentierenden Charakters ferner das Anwenden einer Klassifikation umfasst, die vorhersagt, ob die erfasste Lichtintensität diejenige einer Hefe ist.

12. Verfahren nach Anspruch 11, wobei, wenn die erfasste Lichtintensität nicht diejenige einer Hefe ist, das Bestimmen des Gram-Typs und des fermentierenden Charakters ferner das Anwenden einer Klassifikation umfasst, die vorhersagt, ob die Lichtintensität diejenige eines gramnegativen und fermentierenden Bakterienstamms oder eines gramnegativen und nicht fermentierenden Bakterienstamms ist.

13. Verfahren nach Anspruch 12, wobei:

- die Klassifikation, die vorhersagt, ob die Lichtintensität diejenige eines grampositiven Bakterienstamms ist, eine Klassifikation ist, die die Lichtintensität der grampositiven Bakterienstämme von der Lichtintensität der Menge, die aus den gramnegativen und nicht fermentierenden Bakterienstämmen, den gramnegativen und fermentierenden Bakterienstämmen und den Hefen besteht, unterscheidet;
- die Klassifikation, die vorhersagt, ob die erfasste Lichtintensität diejenige Hefe ist, eine Klassifikation ist, die die Lichtintensität der Hefen von der Intensität der Menge, die aus den negativen und nicht fermentierenden Bakterienstämmen und den negativen und fermentierenden Bakterienstämmen besteht, unterscheidet;
- die Klassifikation, ob die Lichtintensität diejenige eines gramnegativen und fermentierenden Bakterienstamms oder eines gramnegativen und nicht fermentierenden Bakterienstamms ist, eine Klassifikation ist, die die Lichtintensität der gramnegativen und nicht fermentierenden Bakterienstämme von der Lichtintensität der Gruppe, die aus den gramnegativen und fermentierenden Bakterienstämmen besteht, unterscheidet.

14. Verfahren nach einem der Ansprüche 3 bis 13, wobei jede Klassifikation an hyperspektralen Bildern des Bereichs 390 nm-900 nm und gemäß einem Ansatz erlernt wird, der darin besteht, eine Menge von bei der Klassifikation verwendeten spektralen Kanälen schrittweise zu vergrößern bis zum Erhalten eines vorbestimmten Präzisionsschwellenwerts oder einer vorbestimmten maximalen Anzahl von Kanälen.

15. Verfahren nach Anspruch 3, wobei die erste Klassifikation die Lichtintensitäten nur in Abhängigkeit von dem Wellenlängenbereich 470 nm-500 nm und von dem Wellenlängenbereich 575-645 nm unterscheidet.

16. Verfahren nach Anspruch 4, wobei die zweite Klassifikation die Lichtintensitäten nur in Abhängigkeit von dem Wellenlängenbereich 535 nm-675 nm und von dem Wellenlängenbereich 850 nm-875 nm unterscheidet.

17. Verfahren nach einem der Ansprüche 5 bis 7, wobei die zweite Klassifikation die Lichtintensitäten nur in Abhängigkeit von dem Wellenlängenbereich 415 nm-500 nm und von dem Wellenlängenbereich 535 nm-675 nm unterscheidet.

18. Verfahren nach einem der vorhergehenden Ansprüche, wobei die Lichtintensität über eine Anzahl von spektralen Kanälen kleiner oder gleich 24 erfasst wird.

19. Verfahren nach einem der Ansprüche 5 bis 7, wobei die Lichtintensität über eine Anzahl von spektralen Kanälen kleiner oder gleich 5 für jede der ersten und zweiten Klassifikationen und bevorzugt kleiner oder gleich 4 erfasst wird.

20. Verfahren nach einem der vorhergehenden Ansprüche, wobei das Bestimmen des Gram-Typs und des fermentierenden Charakters in Abhängigkeit von der Lichtintensität jedes Pixels einer Menge von Pixeln der Kolonie ausgeführt wird und wobei der Gram-Typ und der fermentierende Charakter durch eine Mehrheitsabstimmung der Ergebnisse der ersten Detektionen bestimmt werden.

21. Verfahren nach Anspruch 20, wobei die Mehrheitsabstimmung eine Abstimmung mit 70 % der Pixel oder mehr oder eine Abstimmung mit der einfachen Mehrheit ist.

22. System zur Charakterisierung eines Mikroorganismus in Form einer in einem nicht chromogenen und nicht fluoreszierenden Nährmedium kultivierten Kolonie, das aufweist:

- eine Beleuchtung, die dazu ausgestaltet ist, die Kolonie des Mikroorganismus in dem Wellenlängenbereich 390 nm-900 nm zu beleuchten;
- einen Sensor, der dazu ausgestaltet ist, in dem Bereich 390 nm-900 nm ein von der Kolonie des beleuchteten Mikroorganismus reflektiertes oder durch sie hindurch transmittiertes hyperspektrales oder multispektrales Bild zu erfassen; und
- eine Computereinheit, die dazu ausgestaltet ist

  ◦ eine Lichtintensität in dem Bereich 390 nm-900 nm in Abhängigkeit von mindestens einem Pixel des hyperspektralen oder multispektralen Bilds der Kolonie des Mikroorganismus zu bestimmen; und
∘ den Mikroorganismus in Abhängigkeit von der in dem Bereich erfassten Lichtintensität als eine Hefe oder ein Bakterienstamm zu bestimmen.

23. System nach Anspruch 22, das dazu ausgestaltet ist, ein Verfahren nach einem der Ansprüche 2 bis 21 zu implementieren.

24. System nach Anspruch 22 oder 23, das dazu ausgestaltet ist, eine Probe, die eine Kolonie von Mikroorganismen und ein Nährmedium, auf dem die Kolonie gewachsen ist, umfasst, insbesondere eine Petri-Schale, zu beleuchten und Bild von ihr zu erfassen.


**Claims**

1. A method for characterizing a microorganism forming a colony cultured in a nonchromogenic and nonfluorescent culture medium, comprising:

   - illuminating, in the wavelength range 390nm-900nm, the colony of the microorganism having a natural electromagnetic response in said range;
   - acquiring, in said range, a luminous intensity reflected by, or transmitted through, said illuminated colony of the microorganism, the luminous intensity acquisition comprising the acquisition of a hyperspectral or multispectral image, and the luminous being determined based on at least one pixel of said image corresponding to the colony; and
   - determining the microorganism as being a yeast or a bacterial strain, depending on the luminous intensity acquired in said range.

2. The method as claimed in claim 1, further comprising detection of the Gram type and of the fermenting character of a bacterial strain, comprising:

   - illuminating, in the wavelength range 390nm-900nm, at least one bacterium of said strain having a natural electromagnetic response in said range;
   - acquiring, in said range, a luminous intensity reflected by, or transmitted through, said illuminated bacterium; and

   determining the Gram type and the fermenting character of the bacterial strain depending on the luminous intensity acquired in said range.

3. The method as claimed in claim 2, in which determination of the Gram type and fermenting character comprises application of a classification predicting whether the luminous intensity acquired is that of a bacterial strain of the Gram-negative type and fermenting.

4. The method as claimed in claim 2 or 3, in which determination of the Gram type and fermenting character comprises application of a classification predicting whether the luminous intensity acquired is that of a bacterial strain of the Gram-positive type or application of a classification predicting whether the luminous intensity acquired is that of a bacterial strain of the Gram-negative type.

5. The method as claimed in claim 3, in which:

   - illumination and acquisition are carried out directly on a sample comprising a colony of the bacterial strain and a culture medium on which said colony has grown, the culture medium being a blood agar, in particular a sheep blood columbia;
   - if the luminous intensity acquired is not that of a bacterial strain of the Gram-negative type and fermenting, determination of the Gram type and fermenting character further comprises application of a classification predicting whether the luminous intensity acquired is that of a bacterial strain of the Gram-positive type.

6. The method as claimed in claim 5, in which, if the luminous intensity acquired is not that of a bacterial strain of the Gram-positive type, determination of the Gram type and fermenting character further comprises application of a classification predicting whether the luminous intensity acquired is that of a yeast.

7. The method as claimed in claim 6, in which

- the classification predicting whether the luminous intensity acquired is that of a bacterial strain of the Gram-negative type and fermenting is a classification distinguishing the luminous intensity of the bacterial strains of the Gram-negative type and fermenting from the luminous intensity of the set made up of the bacterial strains of the negative type and nonfermenting, bacterial strains of the Gram-positive type and yeasts;
- the classification predicting whether the luminous intensity acquired is that of a bacterial strain of the Gram-positive type is a classification distinguishing the luminous intensity of the bacterial strains of the Gram-positive type from the luminous intensity of the set made up of the bacterial strains of the negative type and nonfermenting and yeasts.
- the classification predicting whether the luminous intensity acquired is that of a yeast is a classification distinguishing the luminous intensity of the yeasts from the luminous intensity of the set made up of the bacterial strains of the Gram-negative type and nonfermenting.

8. The method as claimed in claim 2, in which, if the luminous intensity acquired is not that of a yeast, determination of the Gram type and fermenting character further comprises application of a classification predicting whether the luminous intensity acquired is that of a bacterial strain of the Gram-positive type.

9. The method as claimed in claim 8, in which, if the luminous intensity acquired is not that of a bacterial strain of the Gram-positive type, determination of the Gram type and fermenting character further comprises application of a classification predicting whether the luminous intensity is that of a bacterial strain of the Gram-negative type and fermenting or of a bacterial strain of the Gram-negative type and nonfermenting.

10. The method as claimed in claim 9, in which:

- the classification predicting whether the luminous intensity acquired is that of a yeast is a classification distinguishing the luminous intensity of the yeasts from the luminous intensity of the set made up of the bacterial strains of the Gram-positive type, the bacterial strains of the Gram-negative type and nonfermenting and the bacterial strains of the Gram-negative type and fermenting;
- the classification predicting whether the luminous intensity is that of a bacterial strain of the Gram-positive type is a classification distinguishing the luminous intensity of the bacterial strains of the Gram-positive type from the luminous intensity of the set made up of the bacterial strains of the Gram-negative type and nonfermenting and the bacterial strains of the Gram-negative type and fermenting;
- the classification whether the luminous intensity is that of a bacterial strain of the Gram-negative type and fermenting or of a bacterial strain of the Gram-negative type and nonfermenting is a classification distinguishing the luminous intensity of the bacterial strains of the Gram-negative type and nonfermenting from the luminous intensity of the group made up of the bacterial strains of the Gram-negative type and fermenting;

11. The method as claimed in claim 4, in which:

- illumination and acquisition are carried out directly on a sample comprising a colony of the bacterial strain and a culture medium on which said colony has grown, the culture medium being a tryptone-soy agar;
- if the luminous intensity acquired is not that of a bacterial strain of the Gram-positive type, determination of the Gram type and fermenting character further comprises application of a classification predicting whether the luminous intensity acquired is that of a yeast.

12. The method as claimed in claim 11, in which, if the luminous intensity acquired is not that of a yeast, determination of the Gram type and fermenting character further comprises application of a classification predicting whether the luminous intensity is that of a bacterial strain of the Gram-negative type and fermenting or of a bacterial strain of the Gram-negative type and nonfermenting.

13. The method as claimed in claim 12, in which:

- the classification predicting whether the luminous intensity is that of a bacterial strain of the Gram-positive type is a classification distinguishing the luminous intensity of the bacterial strains of the Gram-positive type from the luminous intensity of the set made up of the bacterial strains of the Gram-negative type and nonfermenting, the bacterial strains of the Gram-negative type and fermenting and the yeasts;
- the classification predicting whether the luminous intensity acquired is that of yeast is a classification distinguishing the luminous intensity of the yeasts from the intensity of the set made up of the bacterial strains of the negative type and nonfermenting and the bacterial strains of the negative type and fermenting;

- the classification whether the luminous intensity is that of a bacterial strain of the Gram-negative type and fermenting or of a bacterial strain of the Gram-negative type and nonfermenting is a classification distinguishing the luminous intensity of the bacterial strains of the Gram-negative type and nonfermenting from the luminous intensity of the group made up of the bacterial strains of the Gram-negative type and fermenting.

14. The method as claimed in one of claims 3 to 13, in which each classification is trained on hyperspectral images in the range 390nm-900 nm and according to an approach consisting of increasing step-by-step a set of spectral channels used in the classification until a threshold of predetermined accuracy or a predetermined maximum number of channels is obtained.

15. The method as claimed in claim 3, in which the first classification distinguishes the luminous intensities as a function of the wavelength range 470nm-500nm and of the wavelength range 575-645 nm only.

16. The method as claimed in claim 4, in which the second classification distinguishes the luminous intensities as a function of the wavelength range 535nm - 675 nm and of the wavelength range 850 nm-875 nm only.

17. The method as claimed in one of claims 5 to 7, in which the second classification distinguishes the luminous intensities as a function of the wavelength range 415nm-500nm and of the wavelength range 535 nm-675 nm only.

18. The method as claimed in any one of the preceding claims, in which the luminous intensity is acquired on a number of spectral channels less than or equal to 24.

19. The method as claimed in any one of claims 5 to 7, in which the luminous intensity is acquired on a number of spectral channels less than or equal to 5 for each of the first and second classifications, and preferably less than or equal to 4.

20. The method as claimed in any one of the preceding claims, in which determination of the Gram type and fermenting character is carried out as a function of the luminous intensity of each pixel of a set of pixels of the colony, and in which the Gram type and the fermenting character are determined by a majority vote of the results of said first detections.

21. The method as claimed in claim 20, in which the majority vote is a vote at 70% of the pixels or more or a vote with a simple majority.

22. A system for characterization of a microorganism forming a colony cultured in a nonchromogenic and nonfluorescent culture medium, comprising:

- illumination configured for illuminating the colony of the microorganism in the wavelength range 390nm-900 nm;
- a sensor configured for acquiring, in the range 390nm-900nm, a hyperspectral or multispectral image reflected by, or transmitted through, said illuminated colony of the microorganism; and
- a computer unit configured for:

  ○ determining a luminous intensity in the range 390nm-900nm based on at least one pixel of said image corresponding to the colony; and
  ○ determining the microorganism as being a yeast or a bacterial strain depending on the luminous intensity acquired in said range.

23. The system as claimed in claim 22, configured for implementing a method as claimed in one of claims 2 to 21.

24. The system as claimed in claim 22 or 23, configured for illuminating, and acquiring the image of, a sample comprising a colony of microorganisms and a culture medium on which said colony has grown, in particular a Petri dish.

**Figure 1**

**Figure 2**

**Figure 3**

40

| Croissance d'une ou plusieurs colonies de levures ou de souches bactériennes sur une gélose dans une boite de Pétri | 42 |

| Eclairage à 400-900 nm de la boite de Pétri ouverte et acquisition d'une image hyperspectrale ou multispectrale (« *HSI*(λ) ») à 390-900 nm de la lumière réfléchie par la boite de Pétri | 44 |

| Prétraitement de l'image de radiance *HSI*(λ) | 46 |

| Transformation de l'image de radiance *HSI*(λ) en une image de réflectance (« Υ(λ) ») | 48 |

| Extraction des zones de pixels (« Col(λ) ») correspondant aux colonies de bactéries. | 50 |

| Prédiction du type de Gram et du caractère fermentant de la colonie en fonction d'au moins un spectre de la zone Col(λ) | 52 |

**Figure 4**

**Figure 5**

80

| Constitution d'une base de données d'images HSI de colonies de bactéries et de levures | — 82 |

| Détermination d'une structure des modèles prédiction | — 84 |

| Sélection parcimonieuse des canaux spectraux | — 86 |

**Figure 6**

$$\Upsilon_{i,j}(\lambda)$$

| Y versus {GP, GNF, GNN} | GP versus {Y, GNF, GNN} | GNF versus {Y,GP, GNN} | GNN versus {Y,GP, GNF} |

90    $S_Y$    92    $S_{GP}$    94    $S_{GNF}$    96    $S_{GNN}$

98

$$Classe = f(S_Y, S_{GP}, S_{GNF}, S_{GNN})$$
$$\text{e.g. } Classe \triangleq argmax(S_Y, S_{GP}, S_{GNF}, S_{GNN})$$

**Figure 7A**

**Figure 7B**

**Figure 7C**

| Espèce | code Vitek | Classe |
|---|---|---|
| *Citrobacter koseri* | CIT-KOS | GNF |
| *Enterobacter cloacae* | ENT-CLC | GNF |
| *Escherichia coli* | ESH-COL | GNF |
| *Klebsiella pneumoniae* | KLB-PEU | GNF |
| *Morganella morganii* | MOR-MOR | GNF |
| *Enterococcus faecalis* | ENC-FAE | GP |
| *Staphylococcus aureus* | STA-AUR | GP |
| *Staphylococcus epidermidis* | STA-EPI | GP |
| *Streptococcus agalactiae* | STR-AGA | GP |
| *Streptococcus pyogenes* | STR-PYO | GP |
| *Streptococcus viridans (mitis)* | STR-MIT | GP |
| *Candida albicans* | CAN-ALB | Y |
| *Candida guilliermondii* | CAN-GUI | Y |
| *Candida lusitaniae* | CAN-LUS | Y |
| *Candida tropicalis* | CAN-TRO | Y |
| *Candida parapsilosis* | CAN-PRP | Y |
| *Candida dubliniensis* | CAN-DBL | Y |
| *Candida lusitaniae* | CAN-LUS | Y |
| *Burkholderia cepacia* | BUR-CEP | GNNF |
| *Pseudomonas aeruginosa* | PSD-AEU | GNNF |
| *Stenotrophomonas maltophilia* | STE-MLT | GNNF |

**Figure 8**

|  | Milieu COS | | | |
|---|---|---|---|---|
|  | Bloc 1 | | Bloc 2 | |
| Espèce | Nombre de colonies | Nombre de pixels | Nombre de colonies | Nombre de pixels |
| ACN-BAU | 20 | 1777 | 19 | 1892 |
| ALC-FAE | 2 | 106 | 2 | 92 |
| BUR-CEP | 27 | 810 | 26 | 630 |
| CAN-ALB | 30 | 1525 | 29 | 1255 |
| CAN-DBL | 28 | 1423 | 27 | 1550 |
| CAN-GUI | 51 | 2832 | 51 | 2887 |
| CAN-KEF | 26 | 3434 | 26 | 3830 |
| CAN-KRU | 11 | 855 | 11 | 806 |
| CAN-LUS | 25 | 1556 | 25 | 1581 |
| CAN-PRP | 29 | 1936 | 28 | 2184 |
| CAN-TRO | 21 | 2379 | 21 | 2358 |
| CIT-KOS | 19 | 3135 | 19 | 3316 |
| ENC-FAE | 59 | 4467 | 59 | 4527 |
| ENT-CLC | 9 | 1635 | 9 | 1696 |
| ESH-COL | 10 | 1424 | 10 | 1936 |
| KLB-PEU | 6 | 3238 | 5 | 3143 |
| MOR-MOR | 5 | 4549 | 5 | 2360 |
| PSD-AEU | 11 | 2035 | 10 | 2284 |
| STA-AUR | 15 | 1458 | 14 | 1402 |
| STA-EPI | 17 | 964 | 16 | 730 |
| STE-MLT | 21 | 1686 | 20 | 1684 |
| STR-AGA | 54 | 2264 | 53 | 1758 |
| STR-MIT | 49 | 1531 | 49 | 1616 |
| STR-PYO | 77 | 1548 | 76 | 1654 |
| TOTAL | 622 | 48567 | 610 | 47171 |

**Figure 9**

| Espèce | Milieu TSA | | | |
| | Bloc 1 | | Bloc 2 | |
| | Nombre de colonies | Nombre de pixels | Nombre de colonies | Nombre de pixels |
|---|---|---|---|---|
| ACN-BAU | 8 | 542 | 7 | 476 |
| ALC-FAE | 2 | 73 | 2 | 76 |
| BUR-CEP | 14 | 418 | 13 | 373 |
| CAN-ALB | 6 | 623 | 5 | 529 |
| CAN-DBL | 6 | 390 | 5 | 372 |
| CAN-GUI | 23 | 2102 | 22 | 2191 |
| CAN-KEF | 14 | 2382 | 14 | 1676 |
| CAN-KRU | 33 | 807 | 32 | 775 |
| CAN-LUS | 24 | 2140 | 24 | 2074 |
| CAN-PRP | 21 | 1454 | 20 | 1083 |
| CAN-TRO | 6 | 1457 | 5 | 1378 |
| CIT-KOS | 8 | 1816 | 8 | 1976 |
| ENC-FAE | 36 | 1297 | 35 | 1448 |
| ENT-CLC | 24 | 3129 | 24 | 2455 |
| ESH-COL | 2 | 251 | 2 | 390 |
| KLB-PEU | 2 | 2144 | 1 | 1220 |
| MOR-MOR | 7 | 1151 | 7 | 1359 |
| PSD-AEU | 3 | 355 | 3 | 414 |
| STA-AUR | 23 | 1619 | 22 | 1298 |
| STA-EPI | - | - | - | - |
| STE-MLT | 22 | 894 | 21 | 1078 |
| STR-AGA | 12 | 831 | 12 | 702 |
| STR-MIT | 8 | 140 | 7 | 125 |
| STR-PYO | 24 | 1121 | 23 | 950 |
| TOTAL | 328 | 27136 | 314 | 24418 |

**Figure 10**

| | | Classe prédite | | | | | |
| | | A | B | C | D | TOTAL | Sensibilité |
|---|---|---|---|---|---|---|---|
| Classe réelle | A | $n_{AA}$ | $n_{AB}$ | $n_{AC}$ | $n_{AD}$ | $n_{A\cdot}$ | $n_{AA}/n_{A\cdot}$ |
| | B | $n_{BA}$ | $n_{BB}$ | $n_{BC}$ | $n_{BD}$ | $n_{B\cdot}$ | $n_{BB}/n_{B\cdot}$ |
| | C | $n_{CA}$ | $n_{CB}$ | $n_{CC}$ | $n_{CD}$ | $n_{C\cdot}$ | $n_{CC}/n_{C\cdot}$ |
| | D | $n_{DA}$ | $n_{DB}$ | $n_{DC}$ | $n_{DD}$ | $n_{D\cdot}$ | $n_{DD}/n_{D\cdot}$ |
| | TOTAL | $n_{\cdot A}$ | $n_{\cdot B}$ | $n_{\cdot C}$ | $n_{\cdot D}$ | $n_{\cdot\cdot}$ | |
| | Spécificité | $n_{AA}/n_{\cdot A}$ | $n_{BB}/n_{\cdot B}$ | $n_{CC}/n_{\cdot C}$ | $n_{DD}/n_{\cdot D}$ | | |

**Figure 11**

$n_{AA}$ : *Effectif des échantillons de la classe A bien classés en A par le modèle (TP)*
$n_{AB}$ : *Effectif des échantillons de la classe A mal classés en B par le modèle (FN)*
$n_{BA}$ : *Effectif des échantillons de la classe B mal classés en A par le modèle (FP)*
$n_{\cdot A}$ : *Effectif des échantillons de la classe A*
$n_{A\cdot}$ : *Effectif des échantillons classés en A par le modèle*
$n_{\cdot\cdot}$ : *Effectif total*

$$CR| = \frac{n_{AA}+n_{BB}+n_{CC}+n_{ADD}}{n_{\cdot\cdot}} \qquad BCR = \frac{(n_{AA}/n_{A\cdot})+(n_{BB}/n_{B\cdot})+(n_{CC}/n_{C\cdot})+(n_{DD}/n_{D\cdot})}{4} = \frac{\sum(n_{ii}/n_{i\cdot})}{4}$$

**Figure 12**

**Figure 13**

**FULL TREE / COS**

* Rank by WL / branch 1     ※ Rank by WL / branch 2     ▵ Rank by WL / branch 3

| Ref | Method | Tree | PPM | Parsimonious | Hierarchical | FWHM = 20 nm (CWL ± 10 nm) |
|------|--------|------|-----|--------------|--------------|------|
| Part III | FS-SVM | 0 | COS | YES | YES | |

**Figure 14**

| Ref | Method | Tree | PPM | Parsimonious | Hierarchical | Flat | MODEL |
|---|---|---|---|---|---|---|---|
| Part IV-1 | FS-SVM | 0 | TSA | YES | YES | NO | N-3 |

**Figure 15**

**Forward selection SVM - TSA parsimonious - 12/8/11 channels**

**Figure 16**

**Figure 17**

TSA

COS

**Figure 18**

41

**TSA**

**COS**

**Figure 19**

TSA

COS

**Figure 20**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 3227947 A

**Littérature non-brevet citée dans la description**

- **HUISUNG KIM et al.** Development of a multispectral light-scatter sensor for bacterial colonies. *Journal of Biophotonics*, 2016
- **GUILLEMOT MATHILDE et al.** yperspectral imaging for presumptive identification of bacterial colonies on solid chromogenic culture media''. *proceedings of SPIE*, 2016
- **BOSSOON PARK et al.** Hyperspectral microscope imaging methods to classifying gram-positive and gram-negative foodborne pathogenic bacteria''. *Transaction of the American society of agricultural engineers*, 2015
- **GRAUS MATTHEW et al.** Hyperspectral fluorescence microscopy detects autofluorescent factors that can be exploited as a diagnostic method for species differentiation. *Journal of biomedical optics*, 2017